(19) Europäisches Patentamt
European Patent Office
Office européen des brevets

(11) Veröffentlichungsnummer: **0 356 394 A2**

# (12) EUROPÄISCHE PATENTANMELDUNG

(21) Anmeldenummer: **89810611.7**

(22) Anmeldetag: **17.08.89**

(51) Int. Cl.$^5$: **C 09 B 62/20**
C 07 D 239/38, D 06 P 1/382

(30) Priorität: **26.08.88 CH 3173/88**

(43) Veröffentlichungstag der Anmeldung:
**28.02.90 Patentblatt 90/09**

(84) Benannte Vertragsstaaten:
**BE CH DE FR GB IT LI**

(71) Anmelder: **CIBA-GEIGY AG**
**Klybeckstrasse 141**
**CH-4002 Basel (CH)**

(72) Erfinder: **Hoegerle, Karl, Dr.**
**Conrad Ferdinand Meyer-Strasse 54**
**CH-4059 Basel (CH)**

**Lehmann, Urs, Dr.**
**Palmenstrasse 17**
**CH-4055 Basel (CH)**

## (54) Reaktivfarbstoffe, deren Herstellung und Verwendung.

(57) Reaktivfarbstoffe der Formel

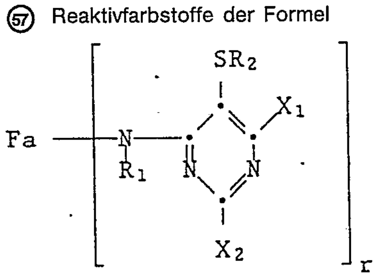

(1),

worin Fa der Rest eines organischen Farbstoffes der Monoazo- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbimid-Reihe, $X_1$ und $X_2$ unabhängig voneinander je Halogen, $R_1$ Wasserstoff oder gegebenenfalls substituiertes $C_1$-$C_4$-Alkyl, $R_2$ gegebenenfalls substituiertes $C_1$-$C_4$-Alkyl und r die Zahl 1 oder 2 ist, ergeben auf hydroxylgruppen- oder stickstoffgruppenhaltigen Textilmaterialien Färbungen von guten Echtheiten.

EP 0 356 394 A2

**Beschreibung**

## Reaktivfarbstoffe, deren Herstellung und Verwendung

Die vorliegende Erfindung betrifft neue Reaktivfarbstoffe, Verfahren zu deren Herstellung und deren Verwendung zum Färben oder Bedrucken von Fasermaterialien.

Gegenstand der Erfindung sind Reaktivfarbstoffe der Formel

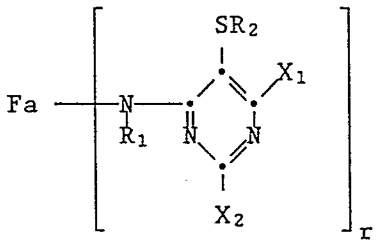

(1),

worin Fa der Rest eines organischen Farbstoffes der Monoazo- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbimid-Reihe, $X_1$ und $X_2$ unabhängig voneinander je Halogen,
$R_1$ Wasserstoff oder gegebenenfalls substituiertes $C_1$-$C_4$-Alkyl,
$R_2$ gegebenenfalls substituiertes $C_1$-$C_4$-Alkyl und r die Zahl 1 oder 2 ist.

Der Rest Fa in Formel (1) kann an seinem Grundgerüst die bei organischen Farbstoffen üblichen Substituenten gebunden enthalten.

Beispiele für geeignete Substituenten im Rest Fa sind: gegebenenfalls durch Sulfo substituiertes $C_1$-$C_4$-Alkyl, womit generell Methyl, Aethyl, n-oder iso-Propyl oder n-, sec.- oder tert.-Butyl umfasst ist; $C_1$-$C_4$-Alkoxy, worunter generell Methoxy, Aethoxy, n- oder iso-Propoxy oder n-, sec.- oder tert.-Butoxy zu verstehen ist; $C_1$-$C_4$-Alkanoylamino, besonders Acetylamino oder Propionylamino; Benzoylamino; Amino; N-Mono- oder N,N-Di-$C_1$-$C_4$-Alkylamino, wobei das Alkyl z.B. durch -OH, -$OCOCH_3$, -$OSO_3H$, -CN oder Halogen weitersubstituiert sein kann, z.B. Methylamino, Aethylamino, n- oder iso-Propylamino, n-, sec.-oder tert.-Butylamino, N,N-Di-β-hydroxyäthylamino, N,N-Di-β-sulfatoäthylamino, Hydroxypropylamino, β-Sulfatoäthylamino, β-Chloräthylamino, β-Acetyloxyäthylamino; gegebenenfalls durch Sulfo und/oder $C_1$-$C_4$-Alkyl substituiertes Phenylamino; Mono-oder Di-Sulfobenzylamino; $C_1$-$C_4$-Alkoxycarbonyl, z.B. Methoxy- oder Aethoxycarbonyl; $C_1$-$C_4$-Alkylsulfonyl, z.B. Methyl- oder Aethylsulfonyl; Trifluormethyl; Nitro; Cyano; Halogen, worunter generell Fluor, Chlor under Brom zu verstehen ist; Phenyl; Carbamoyl; N-Mono- oder N,N-Di-$C_1$-$C_4$-Alkylcarbamoyl; Sulfamoyl; N-Mono- oder N,N-Di-$C_1$-$C_4$-Alkylsulfamoyl; N-(β-hydroxyäthyl)-sulfamoyl; N,N-Di-(β-hydroxyäthyl)-sulfamoyl; N-Phenylsulfamoyl; Hydroxy; Carboxy; Sulfo; Sulfomethyl; Ureido.

Fa bedeutet vorzugsweise den Rest eines organischen Farbstoffes der Mono-oder Polyazo-, Metallkomplexazo-, Anthrachinon-, Phthalocyanin-, Formazan- oder Dioxazinreihe, welcher durch einen oder mehrere der oben genannten Reste substituiert sein kann.

Besonders bevorzugt enthält der Rest Fa eine oder mehrere Sulfogruppen und kann darüberhinaus durch einen oder mehrere der oben genannten Reste weitersubstituiert sein.

Als $C_1$-$C_1$-Alkyl kommt für $R_1$ z.B. in Betracht: Methyl, Aethyl, Propyl, Isopropyl, Butyl, tert.-Butyl, Isobutyl, sek.-Butyl sowie die entsprechenden Reste, die z.B. durch Halogen, Hydroxy, Cyano, Carboxy, Sulfo, Sulfato, $C_1$-$C_4$-Alkoxycarbonyl oder $C_1$-$C_4$-Alkoxy substituiert sind, wie z.B. Carboxymethyl, β-Carboxyäthyl, Methoxycarbonylmethyl, Ethoxycarbonylmethyl, β-Methoxyäthyl, β-Ethoxyäthyl, β-Chloräthyl, γ-Brompropyl, γ-Chlorpropyl, β-Hydroxyäthyl, β-Hydroxybutyl, β-Cyanäthyl, Sulfomethyl, β-Sulfoäthyl, β-Sulfatoäthyl.

$R_1$ steht bevorzugt für Methyl oder Aethyl und insbesondere für Wasserstoff.

Als $C_1$-$C_4$-Alkyl kommt für $R_2$ z.B. Methyl, Aethyl, Propyl, Isopropyl, Butyl, tert.-Butyl, Isobutyl, sek.-Butyl, sowie die entsprechenden Reste, die z.B. durch Halogen, wie z.B. Fluor, Chlor oder Brom, oder Phenyl substituiert sind, in Betracht.

$R_2$ steht bevorzugt für gegebenenfalls durch Halogen, insbesondere Chlor, oder Phenyl substituiertes Methyl oder Aethyl. Besonders bevorzugt für $R_2$ sind Methyl, Monochlormethyl oder Benzyl.

$X_1$ und $X_2$ sind unabhängig voneinander je Halogen, wie z.B. Fluor, Chlor oder Brom, insbesondere Fluor oder Chlor.

Eine bevorzugte Gruppe von erfindungsgemässen Reaktivfarbstoffen umfasst Verbindungen der Formel (1), worin $X_1$ und $X_2$ jeweils Fluor oder Chlor bedeuten.

Die Reaktivfarbstoffe der Formel (1) können eine oder zwei faserreaktive Gruppen enthalten. Falls r in Formel (1) die Zahl 2 ist, können die beiden Reaktivreste gleich oder verschieden sein; vorzugsweise sind beide Reste gleich.

Besonders bevorzugt sind Reaktivfarbstoffe der Formel (1), worin r die Zahl 1 ist.

Die Verbindungen der Formel (1) sind faserreaktiv. Unter faserreaktiven Verbindungen sind solche zu verstehen, die mit den Hydroxygruppen der Cellulose, den Amino-, Carboxy-, Hydroxy- und Thiolgruppen bei

Wolle und Seide, oder mit den Amino-, und eventuell Carboxygruppen von synthetischen Polyamiden unter Bildung kovalenter chemischer Bindungen zu reagieren vermögen.

Eine bevorzugte Gruppe von erfindungsgemässen Reaktivfarbstoffen umfasst Verbindungen der angegebenen Formel (1), worin Fa der Rest eines organischen Farbstoffes der Mono- oder Polyazo-, Metallkomplexazo-, Anthrachinon-, Phthalocyanin-, Formazan- oder Dioxazinreihe, welcher eine oder mehrere Sulfogruppen trägt und darüberhinaus durch einen oder mehrere der zuvor genannten Reste weitersubstituiert sein kann, ist,
$R_1$ Wasserstoff oder unsubstituiertes oder z.B. durch Halogen, Hydroxy, Cyano, Carboxy, Sulfo, Sulfato, $C_1$-$C_4$-Alkoxycarbonyl oder $C_1$-$C_4$-Alkoxy substituiertes $C_1$-$C_4$-Alkyl darstellt, $X_1$ und $X_2$ unabhängig voneinander je Fluor oder Chlor sind,
$R_2$ gegebenenfalls durch Halogen, insbesondere Chlor, oder Phenyl substituiertes Methyl oder Aethyl und r die Zahl 1 ist.

Eine besonders bevorzugte Ausführungsform der vorliegenden Erfindung betrifft Reaktivfarbstoffe der Formel

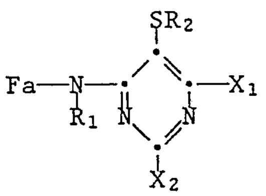 (2),

worin für Fa die zuvor angegebenen Bedeutungen und Bevorzugungen gelten, $R_1$ Wasserstoff, Methyl oder Aethyl ist, $X_1$ und $X_2$ jeweils Chlor oder Fluor bedeuten und $R_2$ Methyl, Monochlormethyl oder Benzyl ist.

Bevorzugte Untergruppen der Reaktivfarbstoffe der Formel (1) sind:

a) Mono- oder Disazofarbstoffe der Formel

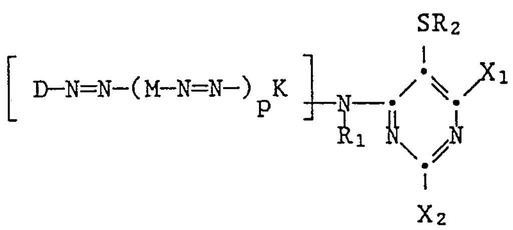 (3)

oder der Formel

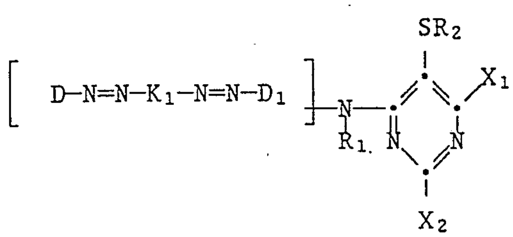 (4),

worin für $R_1$, $R_2$, $X_1$ und $X_2$ jeweils die zuvor angegebenen Bedeutungen und Bevorzugungen gelten, D und $D_1$ unabhängig voneinander eine Diazokomponente der Benzol- oder Naphthalinreihe oder der heterocyclischen Reihe darstellen, M eine Mittelkomponente der Benzol- oder Naphthalinreihe ist, K für eine Kupplungskomponente der Benzol- oder Naphthalinreihe oder der heterocyclischen Reihe steht, $K_1$ der Rest einer Kupplungskomponente der Aminonaphtholsulfonsäure-Reihe ist und p die Zahl 0 oder 1 bedeutet.

Als Untergruppen der Reaktivfarbstoffe der Formel (3) sind zu nennen:

Verbindungen der Formel

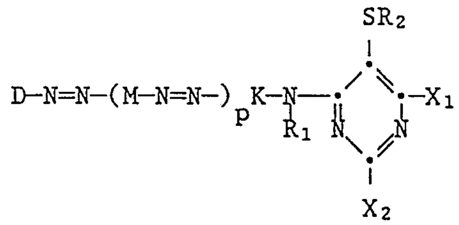 (5)

und Verbindungen der Formel

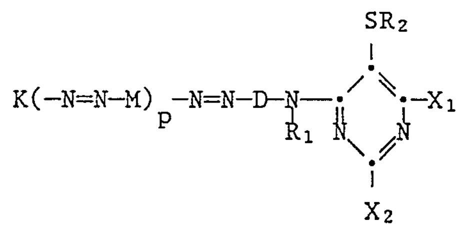 (6),

worin $R_1$, $R_2$, $X_1$, $X_2$, K, D, M und p jeweils die zuvor angegebenen Bedeutung haben.

Die Reste D und $D_1$ können sich z.B. von den folgenden Diazokomponenten ableiten: Aminobenzol, 1-Amino-2-, -3- oder -4-methylbenzol, 1-Amino-2-, -3- oder -4-methoxybenzol, 1-Amino-2-, -3- oder -4-chlorbenzol, 1-Amino-2,5-dichlorbenzol, 1-Amino-2,5-dimethylbenzol, 1-Amino-3-methyl-6-methoxybenzol, 1-Amino-2-methoxy-4-nitrobenzol, 1-Aminobiphenyl, 1-Aminobenzol-2-, -3- oder -4-carbonsäure, 2-Aminodiphenyläther, 1-Aminobenzol-2-, -3- oder -4-sulfonsäureamid, 1-Aminobenzol-2-, -3- oder -4-sulfonsäure, 1-Aminobenzol-2,4- und -2,5-disulfonsäure, 1-Amino-4-methylbenzol-2-sulfonsäure, 1-Amino-4-methoxybenzol-2-sulfonsäure, 1-Amino-4-äthoxybenzol-2-sulfonsäure, 1-Amino-3-methylbenzol-6-sulfonsäure, 1-Amino-6-methylbenzol-3- oder -4-sulfonsäure, 1-Aminonaphthalin, 2-Aminonaphthalin, 1-Aminonaphthalin-2-, -4-, -5-, -6-, -7- oder -8-sulfonsäure, 2-Aminonaphthalin-1-, -3-, -4-, -5-, -6-, -7- oder -8-sulfon säure, 1-Aminonaphthalin-3,6- oder -5,7-disulfonsäure, 2-Aminonaphthalin-1,5-, -1,7-, -3,6-, -5,7-, -4,8- oder -6,8-disulfonsäure, 1-Aminonaphthalin,-2,5,7-trisulfonsäure, 2-Aminonaphthalin-1,5,7-, -3,6,8- oder -4,6,8-trisulfonsäure, 2-Amino-5-methylnaphthalin-1-sulfonsäure, 4-Aminoazobenzol-3,4′-disulfonsäure, 3-Methoxy-4-amino-6-methylazobenzol-2′,4′-disulfonsäure, 3-Methoxy-4-amino-6-methylazobenzol-2′,5′-disulfonsäure, 1-Amino-4-β-sulfatoäthylsulfonylbenzol, 1-Amino-4-vinylsulfonylbenzol, 1-Amino-3-vinylsulfonylbenzol, 1-Amino-4-β-sulfatoäthylsulfonylbenzol-2-sulfonsäure, 1-Amino-4-[β-(β′-chloräthylsulfonyl)äthylaminocarbonyl]benzol-2-sulfonsäure, 1-Amino-4-β-(vinylsulfonyl)äthylaminocarbonylbenzol-2-sulfonsäure, 1-Amino-3-γ-(vinylsulfonyl)butyrylaminobenzol-6-sulfonsäure, 1-Amino-3-vinylsulfonyl-6-methoxybenzol, 1-Amino-3-β-(vinylsulfonyl)äthylaminocarbonyl-6-methoxybenzol und 1-Amino-3-β-(vinylsulfonyl)äthylaminocarbonylbenzol; ferner Diaminobenzole oder Diaminonaphthaline, wie 1,4-Diaminobenzol-2-sulfonsäure, 1,4-Diaminobenzol-2,5-disulfonsäure, 1,4-Diaminobenzol-2,6-disulfonsäure, 1,3-Diaminobenzol-4-sulfonsäure, 1,3-Diaminobenzol-4,6-disulfonsäure, 1,4-Diamino-2-chlorbenzol-5-sulfonsäure, 1,4-Diamino-2-methylbenzol-5-sulfonsäure, 1,5-Diamino-6-methylbenzol-3-sulfonsäure, 1,3-Diamino-6-methylbenzol-4-sulfonsäure, 1,4-Diaminobenzol-2-carbonsäure, 1,3-Diaminobenzol-4-carbonsäure, 1-Amino-3-aminomethylbenzol-6-sulfonsäure, 1-Amino-3-aminomethyl-4-methoxybenzol-2-sulfonsäure, 2,6-Diaminonaphthalin-4,8-disulfonsäure, 2-Amino-5-aminomäthylnaphthalin-1-sulfonsäure und 2-Amino-5-aminomethylnaphthalin-1,7-disulfonsäure; statt eines Diamins kann auch eine Aminoacetylaminoverbindung eingesetzt werden, aus welcher nachträglich die Acetylgruppe durch Verseifen wieder abgespalten wird, z.B. 1-Acetylamino-3-aminobenzol-4-sulfonsäure oder 1-Acetylamino-4-aminobenzol-3-sulfonsäure.

Weiterhin leiten sich D bzw. $D_1$ z.B. von Thienyl, Thiazolyl, Isothiazolyl, 1,2,4-Thiadiazolyl, 1,3,4-Thiadiazolyl, Benzthiazolyl, Benzisothiazolyl, Pyrazolyl, Imidazolyl, 1,2,3-Triazolyl, 1,2,4-Triazolyl, Thiophenyl, Benzthiophenyl, Tetrahydrobenzthiophenyl, Pyridinyl, Indazolyl, ab, wobei jeder der zuvor genannten Reste substituiert sein kann, insbesondere durch $C_1$-$C_8$-Alkyl, $C_1$-$C_8$-Alkoxy, $C_2$-$C_8$-Alkanoylamino, $C_2$-$C_8$-Alkoxycarbonylamino, Benzoylamino, Amino, Mono- oder Dialkylamino mit 1 bis 8 Kohlenstoffatomen im Alkylrest, Phenylamino, $C_2$-$C_8$-Alkoxycarbonyl, Nitro, Cyan, Trifluormethyl, Halogen, Sulfamoyl, am Stick stoffatom ein- oder zweifach durch $C_1$-$C_4$-Alkyl, $C_5$-$C_7$-Cycloalkyl oder Phenyl substituiertes Sulfamoyl, Carbamoyl, Ureido, Hydroxy, $C_1$-$C_8$-Alkylsulfonyl, Phenylsulfonyl, durch $C_1$-$C_4$-Alkyl und/oder Sulfo und/oder Halogen substituiertes Phenylsulfonyl oder Phenoxysulfonyl, Carboxy, Sulfomethyl, Sulfo, Sulfato, Thiosulfato und gegebenenfalls können je 2 benachbarte Substituenten der genannten Ringsysteme weiter ankondensierte Phenylringe oder Cyclohexylringe bilden.

Der Rest M kann sich z.B. von folgenden Mittelkomponenten ableiten:

Anilin; m-Toluidin;

2,5-Dimethyl- oder -Dimethoxy-anilin;

m-Amino-anisol; m-Acetylamino-, m-Propionylamino-, m-Butyrylamino- oder m-Benzoylamino-anilin;
m-Amino-phenylharnstoff;
4-Acetamino-2-amino-toluol oder -anisol;
2-Amino-4-methylanisol;
1-Aminonaphthalin-6- oder -7-sulfonsäure;
2-Amino-4-acetylamino-benzolsulfonsäure;
2-Amino-5-naphthol-7-sulfonsäure;
2-Amino-8-naphthol-6-sulfonsäure;
2-(4-Aminobenzoyl-amino)-5-naphthol-7-sulfonsäure;
Acetoacet-3-sulfo-4-amino-anilid.

Aus der grossen Zahl der möglichen Kupplungskomponenten K und $K_1$ seien die folgenden beispielhaft genannt:

Phenol, 1-Hydroxy-3- oder -4-methylbenzol, 1-Hydroxybenzol-4-sulfonsäure, 1-Hydroxynaphthalin, 2-Hydroxynaphthalin, 2-Hydroxynaphthalin-6-oder -7-sulfonsäure, 2-Hydroxynaphthalin-3,6- oder -6,8-disulfonsäure, 1-Hydroxynaphthalin-4-sulfonsäure, 1-Hydroxynaphthalin-4,6- oder -4,7-disulfonsäure, 1-Amino-3-methylbenzol, 1-Amino-2-methoxy-5-methylbenzol, 1-Amino-2,5-dimethylbenzol, Phenylharnstoff, 3-Aminophenylharnstoff, 1-Amino-3-acetylaminobenzol, 1-Amino-3-hydroxyacetylaminobenzol, 1,3-Diaminobenzol-4-sulfonsäure, 1-Aminonaphthalin-6-oder -8-sulfonsäure, 1-Amino-2-methoxynaphthalin-6-sulfonsäure, 2-Aminonaphthalin-5,7-disulfonsäure, 1-Amino-8-hydroxynaphthalin-4-sulfonsäure, 1-Amino-8-hydroxynaphthalin-6-sulfonsäure, 1-Amino-8-hydroxynaphthalin-2,4-disulfon säure, 2-Hydroxy-3-aminonaphthalin-5,7-disulfonsäure, 1-Amino-8-hydroxynaphthalin-2,4,6-trisulfonsäure, 1-Hydroxy-8-acetylaminonaphthalin-3-sulfonsäure, 1-Benzoylamino-8-hydroxynaphthalin-3,6- oder -4,6-disulfonsäure, 1,7-Dihydroxynaphthalin-3-sulfonsäure, 2-Benzoylamino-5-hydroxynaphthalin-7-sulfonsäure, 2-Amino-5-hydroxynaphthalin-7-sulfonsäure, 2-Methyl-bzw. 2-Aethylamino-5-hydroxynaphthalin-7-sulfonsäure, 2-(N-Acetyl-N-methylamino)-5-hydroxynaphthalin-7-sulfonsäure, 2-Acetylamino-5-hydroxynaphthalin-7-sulfonsäure, 2-Amino-5-hydroxynaphthalin-1,7-disulfonsäure, 2-Amino-8-hydroxynaphthalin-6-sulfonsäure, 2-Methyl-bzw. -Aethylamino-8-hydroxynaphthalin-6-sulfonsäure, 2-(N-Acetyl-N-methylamino)-8-hydroxynaphthalin-6-sulfonsäure, 2-Acetylamino-8-hydroxynaphthalin-6-sulfonsäure, 2-Amino-8-hydroxynaphthalin-3,6-disulfonsäure, 2-Acetylamino-8-hydroxynaphthalin-3,6-disulfonsäure, 1-Amino-5-hydroxynaphthalin-7-sulfonsäure, 1-Amino-8-hydroxynaphthalin-3,6- bzw. -4,6-disulfonsäure, 1-Acetylamino-8-hydroxynaphthalin-3,6- bzw. -4,6-disulfonsäure, 1-(4′-Aminobenzoylamino)-8-hydroxynaphthalin-3,6- bzw. -4,6-disulfonsäure, 1-(4′-Nitrobenzoylamino)-8-hydroxynaphthalin-3,6-bzw. -4,6-disulfonsäure, 1-(3′-Aminobenzoylamino)-8-hydroxynaphthalin-3,6-bzw. -4,6-disulfonsäure, 1-(3′-Nitrobenzoylamino)-8-hydroxynaphthalin-3,6- bzw. -4,6-disulfonsäure, 2-(4′-sulfophenylamino)-5-hydroxynaphthalin-7-sulfonsäure, 3-Methylpyrazolon-(5), 1-Phenyl-3-methyl-5-pyrazolon, 1-(4′-Sulfophenyl)-3-methyl-5-pyrazolon, 1-(4′-Sulfophenyl)pyrazolon-(5)-3-carbonsäure, 1-(3′-Aminophenyl)-3-methyl-5-pyrazolon, 1-(2′,5′-Disulfophenyl)-3-methyl-5-pyrazolon, 1-(2′-Methyl-4′-sulfophenyl)-5-pyrazolon-3-carbonsäure, 1-(4′,8′-Disulfonaphthyl-[2′])-3-methyl-5-pyrazolon, 1-(5′,7′-Disulfonaphthyl-[2′])-3-methyl-5-pyrazolon, 1-(2′,5′-Dichlor-4′-sulfophenyl)-3-methyl-5-pyrazolon, 3-Aminocarbonyl-4-methyl-6-hydroxypyridon-(2), 1-Aethyl-3-cyan- oder -3-chlor-4-methyl-6-hydroxypyridon-(2), 1-Aethyl-3-sulfomethyl-4-methyl-6-hydroxypyridon-(2), 2,4,6-Triamino-3-cyanpyridin, 2-(3′-Sulfophenylamino)-4,6-diamino-3-cyanpyridin, 2-(2′-Hydroxyethylamino)-3-cyan-4-methyl-6-aminopyridin, 2,6-Bis-(2′-hydroxyäthylamino)-3-cyano-4-methylpyridin, 1-Aethyl-3-carbamoyl-4-methyl-6-hydroxypyridon-(2), 1-Aethyl-3-sulfomethyl-4-methyl-5-carbamoyl-6-hydroxypyridon-(2), 1-Aethyl-3-sulfomethyl-4-methyl-6-hydroxypyridon-(2), N-Acetoacetylaminobenzol, 1-(N-Acetoacetylamino)-2-methoxybenzol-5-sulfonsäure, 4-Hydroxychinolon-(2), 1-Amino-8-hydroxy-2-(phenylazo)-naphthalin-3,6-disulfonsäure, 1-Amino-8-hydroxy-2-(4′-sulfophenylazo)-naphthalin-3,6-disulfonsäure, 1-Amino-8-hydroxy-2-(2′,5′-di sulfophenylazo)-naphthalin-3,6-di-sulfonsäure, 1-β-Aminoäthyl-3-cyan-4-methyl-6-hydroxypyridon-(2), 1-γ-Aminopropyl-3-sulfomethyl-4-methyl-6-hydroxypyridon-(2), 1,3-Diaminobenzol, 1-Amino-3-N,N-di-β-hydroxyäthylaminobenzol, 1-Amino-3-N,N-di-β-sulfatoäthylaminobenzol, 1-Amino-3-N,N-di-β-hydroxyäthylamino-4-methoxybenzol, 1-Amino-3-N,N-di-β-sulfato-äthylamino-4-methoxybenzol, 1-Amino-3-sulfo-benzylamino-benzol, 1-Amino-3-sulfo-benzylamino-4-chlorbenzol, 1-Amino-3-N,N-di-sulfo-benzylaminobenzol, N,N-Di-(β-sulfatoäthyl)-anilin, 3-Acetylamino-N,N-di-(β-sulfatoäthyl)-anilin, 3-Methyl-N,N-di-(β-sulfatoäthyl)anilin, N-Aethyl-N-(β-hydroxyäthyl)-anilin, N-Aethyl-N-(β-acetoxyäthyl)-anilin, 3-Acetylamino-N,N-di(β-hydroxyäthyl)-anilin, 3-Methyl-N,N-di-(β-acetoxyäthyl)-anilin, 2-Methoxy-5-acetylamino-N-(β-acetoxyäthyl)-N-benzylanilin, 2-Chlor-5-acetylamino-N-(γ-phenoxy-β-hydroxy-n-propyl)-anilin, 3-Ureidoanilin, N-Aethyl-N-(3′-sulfobenzyl)-anilin, 3-Methyl-N-äthyl-N-(β-sulfatoäthyl)anilin, 3-Methyl-N,N-di-(β-hydroxyäthyl)-anilin, 3-Methyl-6-methoxy-N,N-di-(β-hydroxyäthyl)-anilin.

Entsprechen die erfindungsgemässen Reaktivfarbstoffe der Formel (3) bzw. (4) und handelt es sich demgemäss bei $Fa\{NR_1-]_r$ in Formel (1), wobei $R_1$ und r die unter Formel (1) angegebenen Bedeutungen haben, um den Rest einer Mono- oder Disazoverbindung, so kann sich dieser z.B. von den folgenden organischen Mono- oder Disazofarbstoffen ableiten:

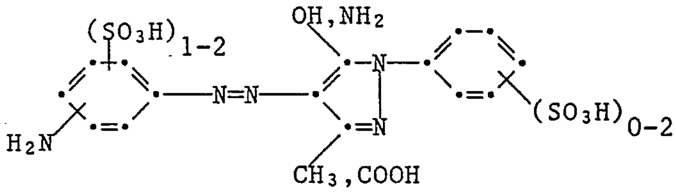

(7)

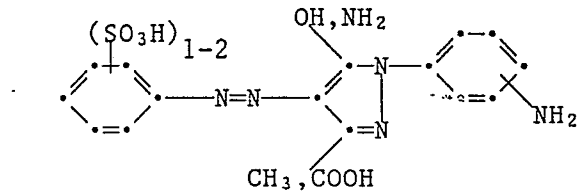

(8)

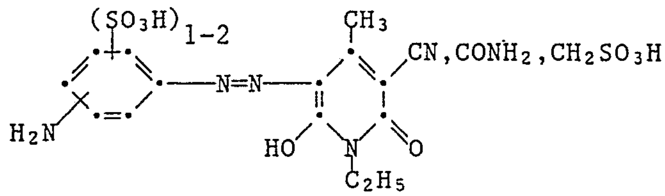

(9)

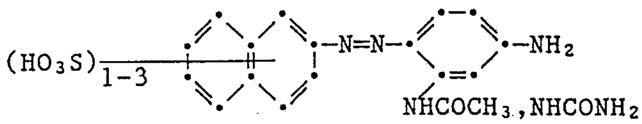
(10)

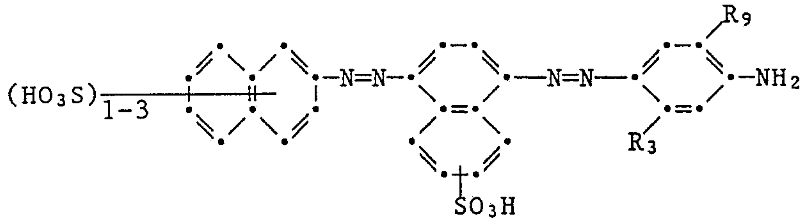
(11)

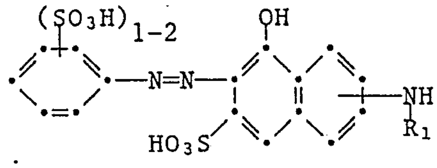
(12)

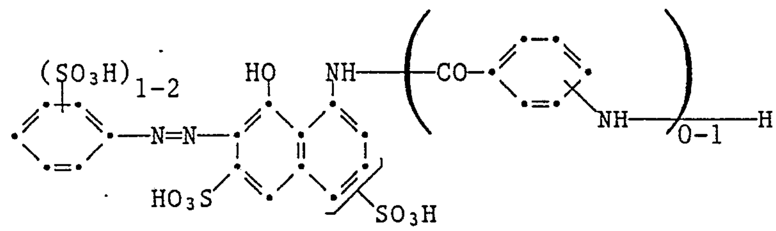
(13)

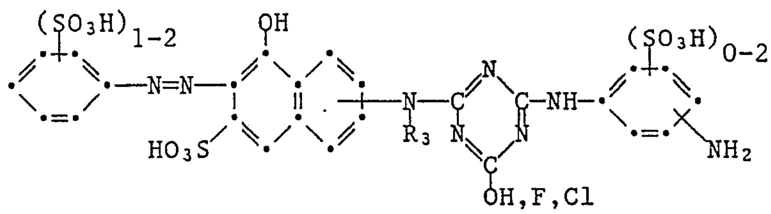
(14)

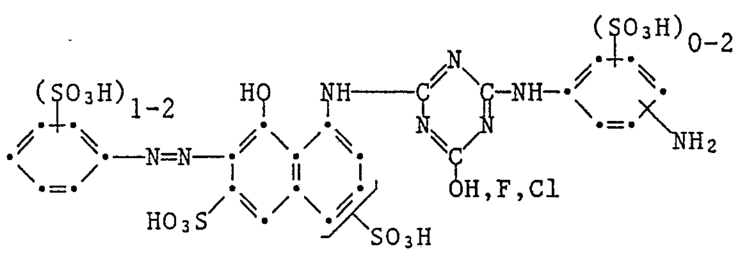
(15)

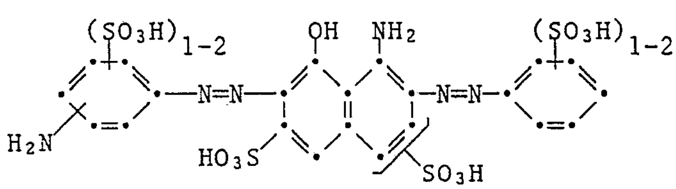
(16)

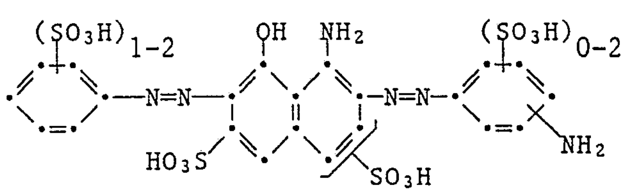
(17)

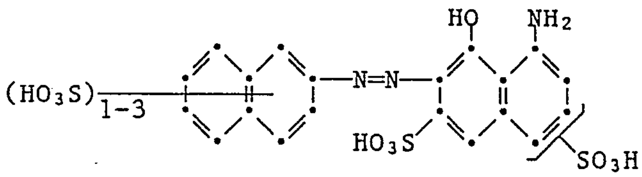

(18)

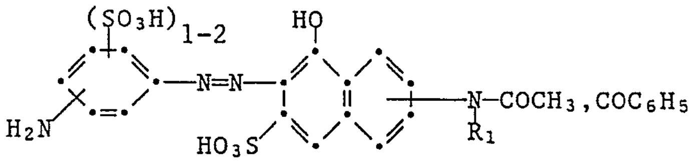

(19)

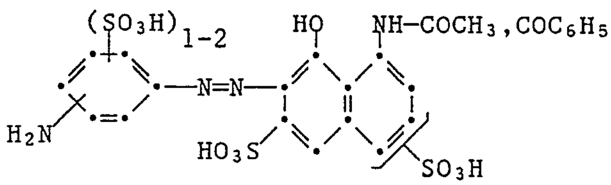

(20)

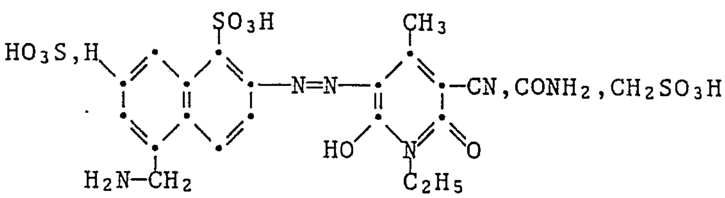

(21)

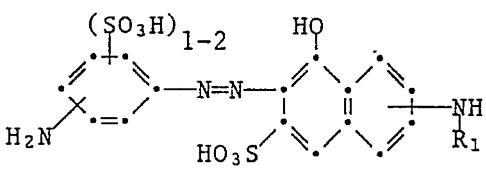

(22)

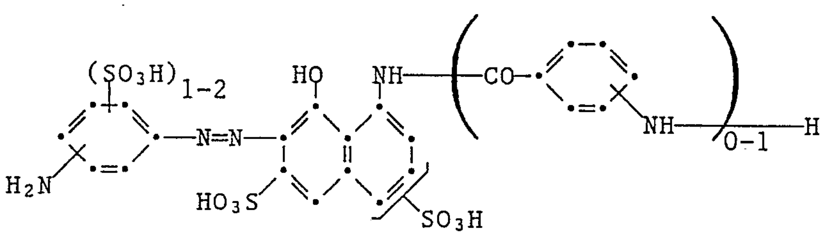

(23)

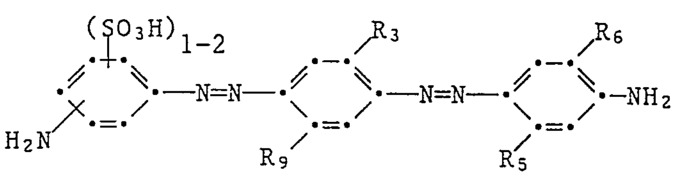

(24)

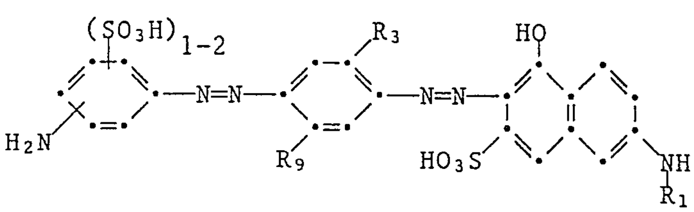

(25)

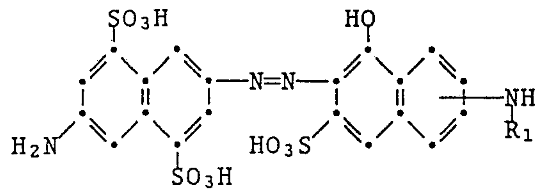

(26)

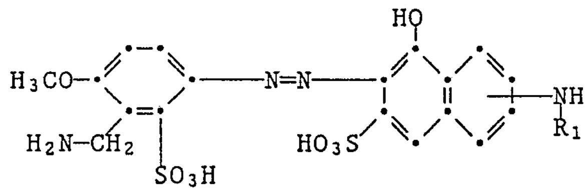

(27)

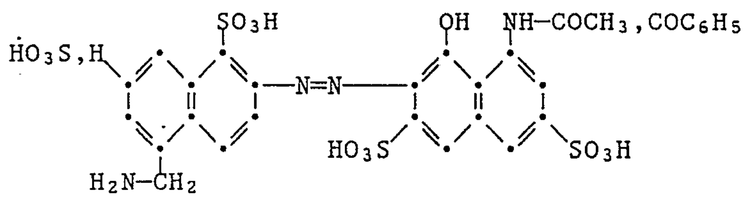

(28)

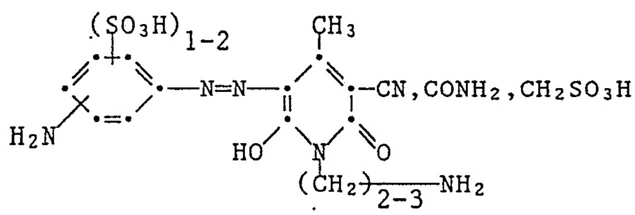

(29)

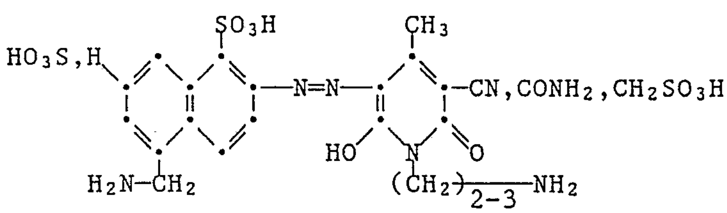

(30)

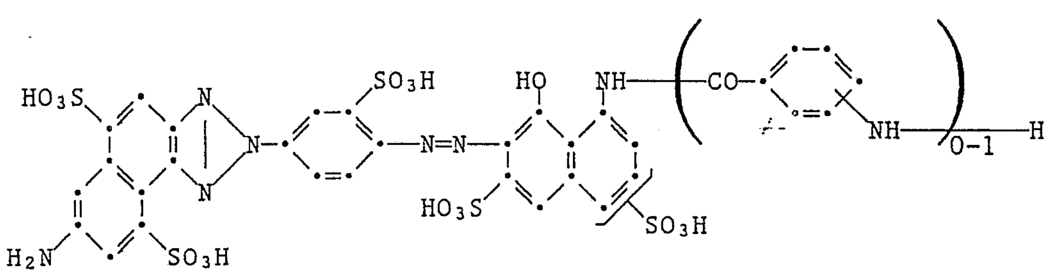

(31)

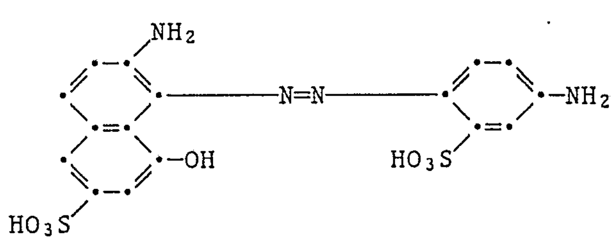

(32)

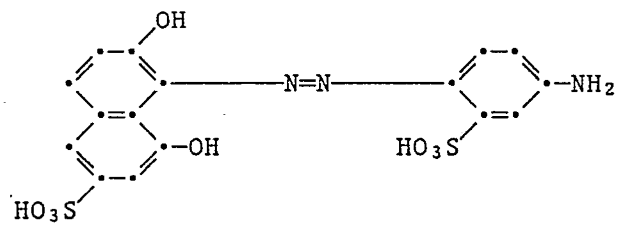

(33)

In den Formeln (7) bis (33) bedeuten $R_3$, $R_5$, $R_6$ und $R_9$ unabhängig voneinander z.B. Wasserstoff, $C_1$-$C_4$-Alkyl, $C_1$-$C_4$-Alkoxy, $C_1$-$C_4$-Alkanoylamino, Ureido oder Halogen und $R_1$ hat die unter Formel (1) angegebenen Bedeutungen.

b) Metallkomplexe von Mono- oder Disazofarbstoffen der zuvor angegebenen Formeln (3) und (4), welche zur Metallkomplexbildung befähigte Gruppen, z.B. Hydroxy, Carboxy, Amino oder Sulfo, aufweisen.

Handelt es sich bei $Fa{+}NR_1{-}]_r$ in Formel (1), wobei $R_1$ und r die unter Formel (1) angegebenen Bedeutungen haben, um den Rest eines Metallkomplexazofarbstoffes, so kann sich dieser Rest z.B. von einem Metallkomplex der im folgenden genannten Farbstoffe ableiten:

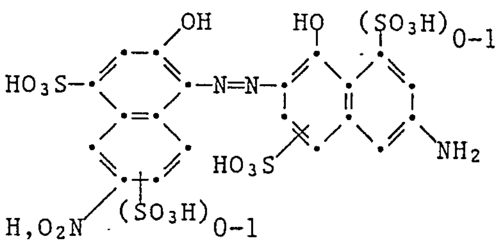

(34)

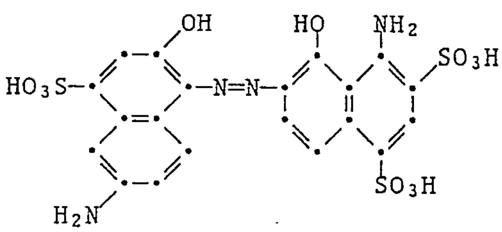

(35)

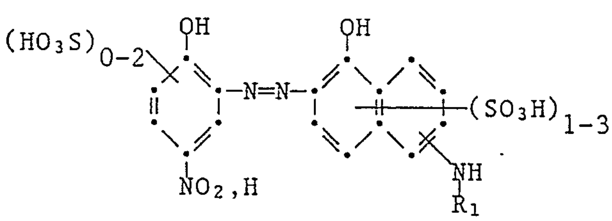

(36)

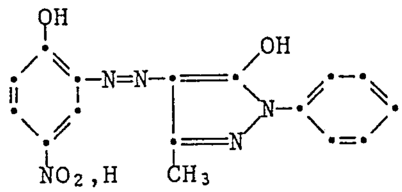

(37)

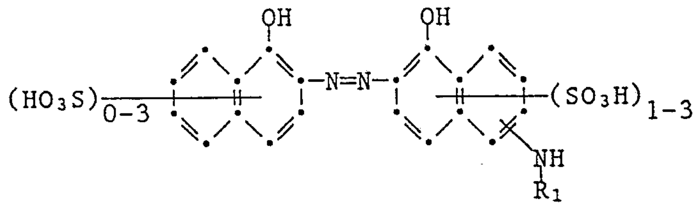

(38)

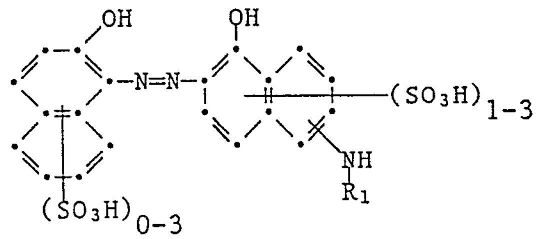

(39)

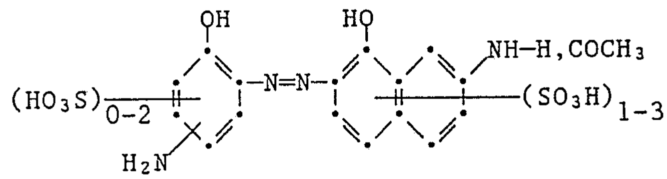

(40),

wobei $R_1$ die unter Formel (1) angegebene Bedeutung hat.

Als Metallatome sind Cu und Cr (1:1-Komplexe) oder Cr und Co (1:2-Komplexe) bevorzugt. Cr- und Co-Komplexe können die Azoverbindungen der Formeln (34) bis (40) einmal oder zweimal enthalten, d.h. sie können symmetrisch oder mit beliebigen anderen Liganden unsymmetrisch aufgebaut sein.

Beispiele für geeignete Kupferkomplexfarbstoffe, die dem Rest $Fa{+}NR_1{-}]_r$ in Formel (1), wobei $R_1$ und r die unter Formel (1) angegebenen Bedeutungen haben, zugrunde liegen können, sind:

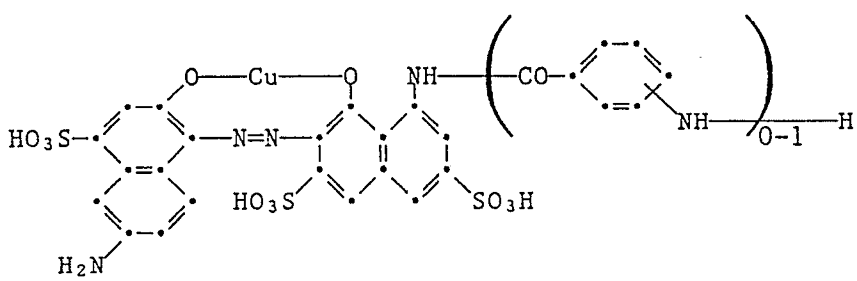

(41)

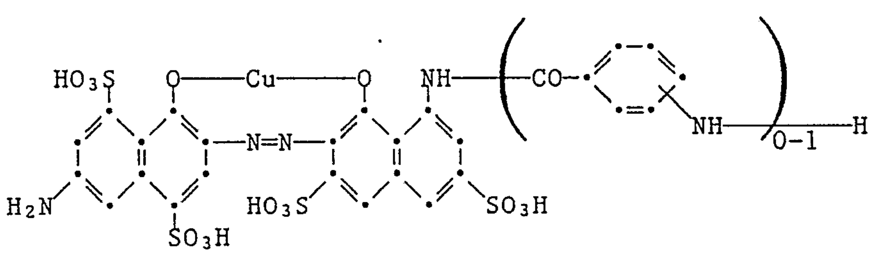

(42)

c) Anthrachinonfarbstoffe der Formel

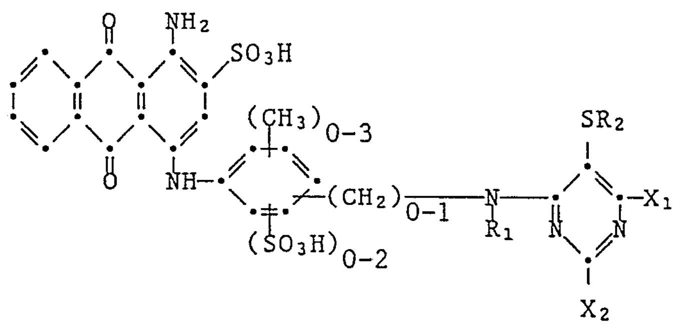

(43),

worin $R_1$, $R_2$, $X_1$ und $X_2$ die unter Formel (1) angegebenen Bedeutungen haben.

d) Formazanfarbstoffe der Formel

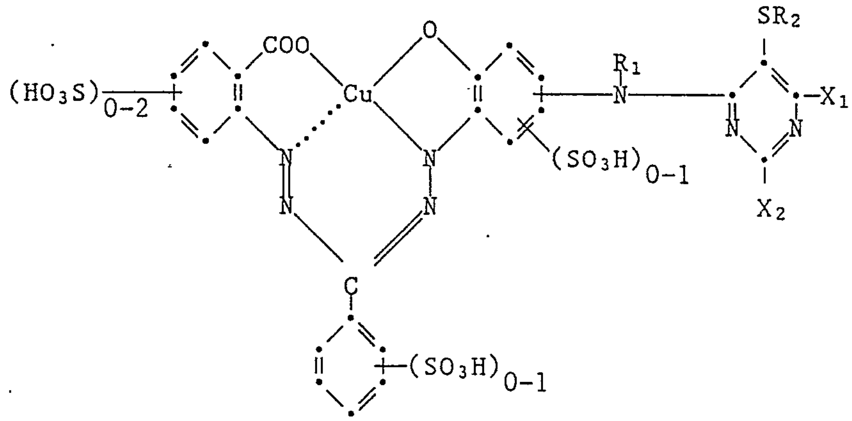

(44),

worin $R_1$, $R_2$, $X_1$ und $X_2$ die unter Formel (1) angegebenen Bedeutungen haben.

e) Phthalocyaninfarbstoffe der Formel

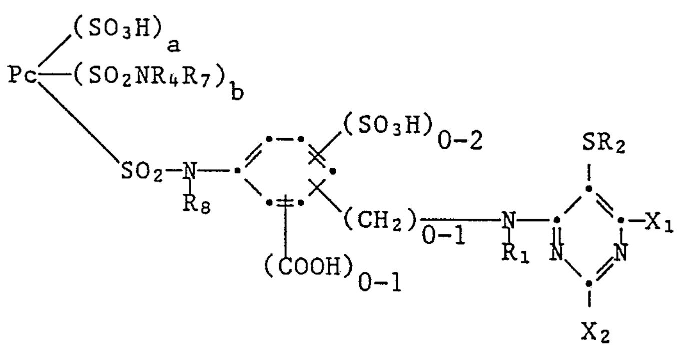

(45),

worin $R_1$, $R_2$, $X_1$ und $X_2$ die unter Formel (1) angegebenen Bedeutungen haben, Pc der Rest eines Cu- oder Ni-Phthalocyanins ist, $R_4$, $R_7$ und $R_8$ unabhängig voneinander $C_1$-$C_4$-Alkyl und insbesondere Wasserstoff bedeuten und a und b ganze oder gebrochene Zahlen von 1 bis 3 sind, wobei a und b zusammen 3,0 ergeben.

f) Reaktivfarbstoffe der Formel (1), worin $Fa{+}NR_1]_r$, wobei $R_1$ und r die unter Formel (1) angegebenen Bedeutungen haben, ein Rest eines Dioxazinfarbstoffs der Formel

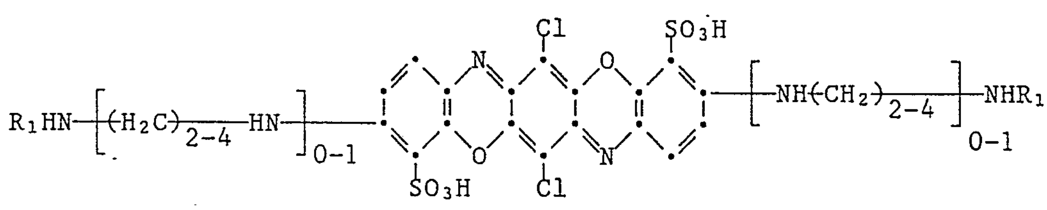

(46),

worin $R_1$ die unter Formel (1) angegebene Bedeutung hat, ist.

Ganz besonders bevorzugte Reaktivfarbstoffe der vorliegenden Erfindung sind:

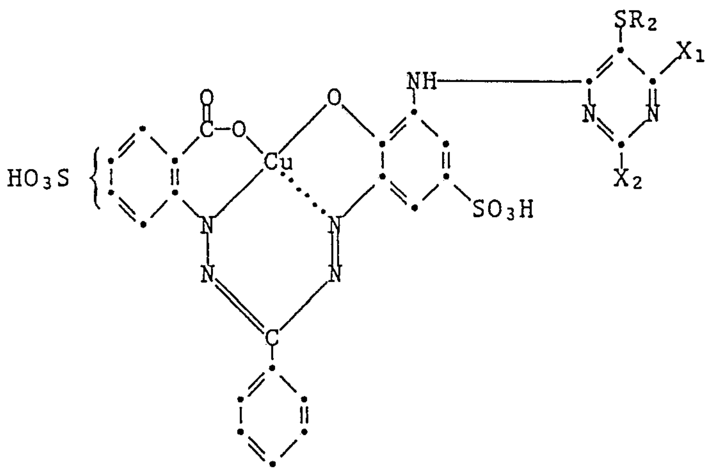

(47)

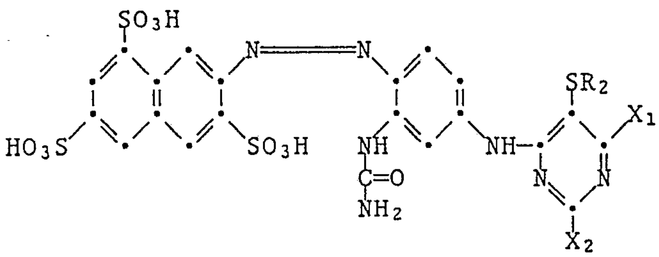

(48)

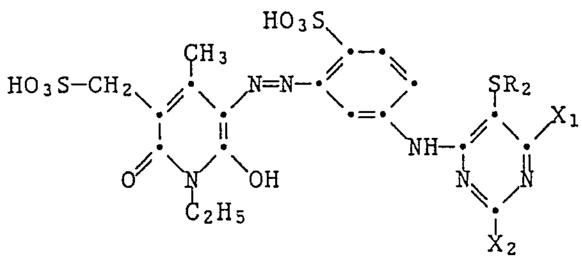

(49)

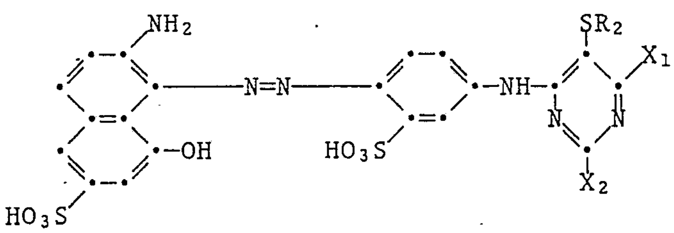

(50)

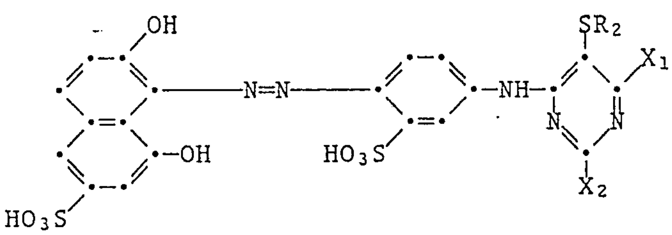

(51)

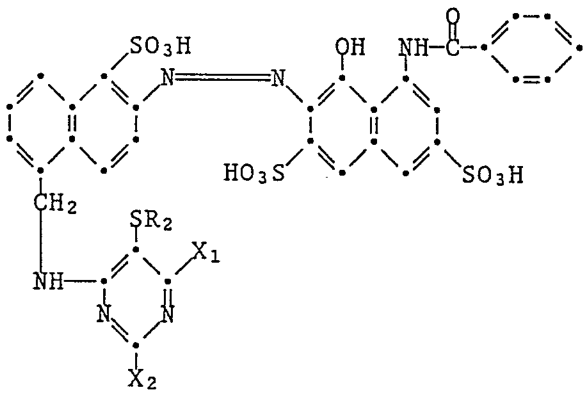

(52),

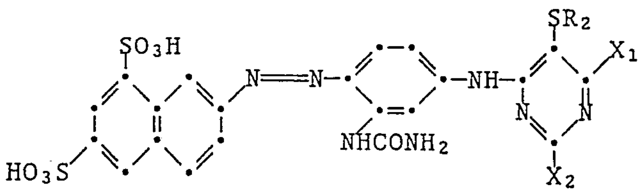

(53),

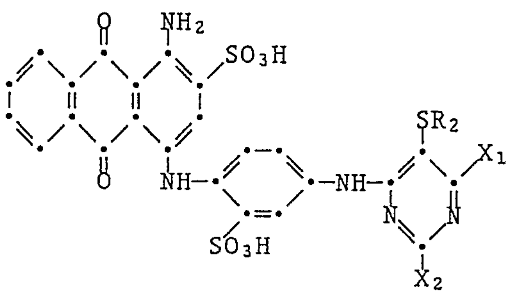

(54),

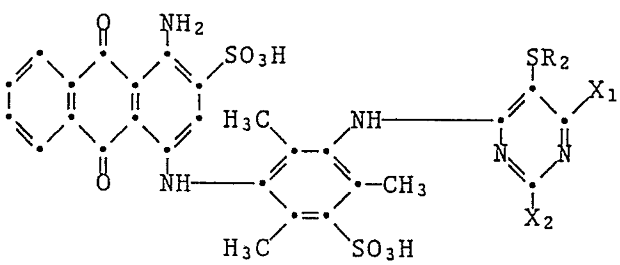

(55),

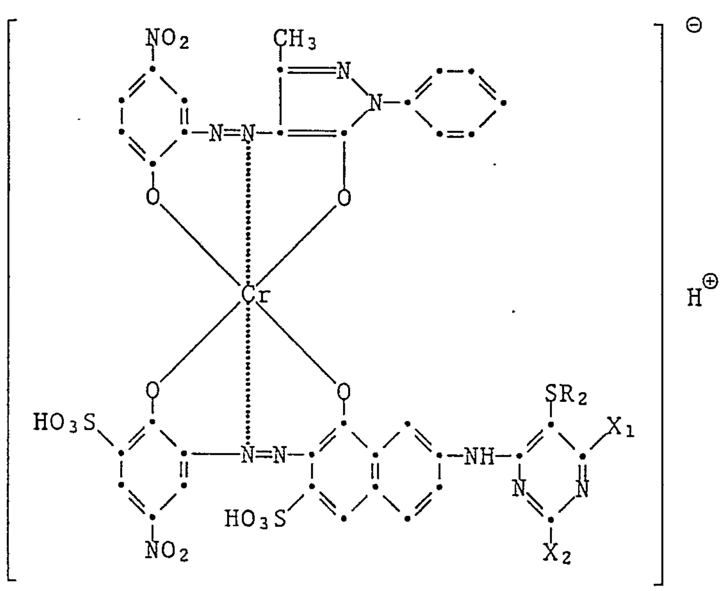

(56),

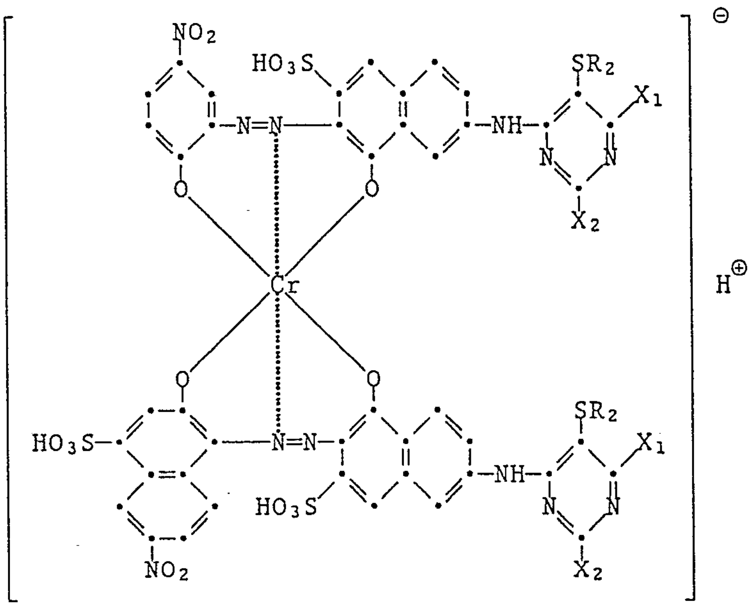

(57),

worin $R_2$, $X_1$ und $X_2$ die unter Formel (1) angegebenen Bedeutungen haben.

Ganz besonders wertvoll sind Reaktivfarbstoffe der Formeln (47) bis (57), in denen $X_1$ und $X_2$ jeweils Fluor oder Chlor und $R_2$ gegebenenfalls durch Halogen, insbesondere Chlor, oder Phenyl substituiertes Methyl ist.

Gegenstand der Erfindung ist ferner ein Verfahren zur Herstellung der erfindungsgemässen Reaktivfarbstoffe, welches dadurch gekennzeichnet ist, dass man z.B. organische Farbstoffe der Formel

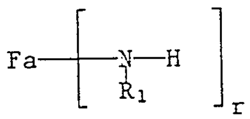

(58)

mit mindestens einem Aequivalent eines Pyrimidinderivats der Formel

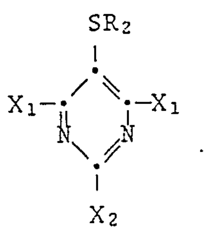

(59)

zu einem Reaktivfarbstoff der Formel (1) kondensiert, wobei Fa, $R_1$, $R_2$, $X_1$, $X_2$ und r die unter Formel (1) angegebenen Bedeutungen haben.

Die Kondensation des Farbstoffes der Formel (58) mit einem Pyrimidinderivat der Formel (59) erfolgt vorzugsweise in wässriger Lösung, Emulsion oder Suspension, bei niedriger Temperatur, z.B. 0 bis 40°C, und bei schwach saurem, neutralem bis schwach alkalischem pH-Wert. Vorteilhaft wird der bei der Kondensation freiwerdende Halogenwasserstoff laufend durch Zugabe wässriger Alkalihydroxide, -carbonate, -bicarbonate oder -acetate neutralisiert.

Die wichtigsten Ausführungsformen sind in den Beispielen dargestellt.

Die Verbindungen der Formel (58) sind an sich bekannt und können in Analogie zu bekannten Verbindungen hergestellt werden.

Die Verbindungen der Formel (59) sind zum Teil bekannt und können in Analogie zu bekannten Verbindungen hergestellt werden.

Als geeignete Pyrimidinverbindungen der Formel (59) seien zum Beispiel genannt:

2,4,6-Trifluor-5-methylthiopyrimidin,
2,4,6-Trichlor-5-methylthiopyrimidin,
2,4,6-Tribrom-5-methylthiopyrimidin,
2,4,6-Trifluor-5-äthylthiopyrimidin,
2,4,6-Trichlor-5-äthylthiopyrimidin,
2,4,6-Tribrom-5-äthylthiopyrimidin,
2,4,6-Trifluor-5-chloromethylthiopyrimidin,

2,4,6-Trichlor-5-chloromethylthiopyrimidin,
2,4,6-Tribrom-5-chloromethylthiopyrimidin,
2,4,6-Trifluor-5-β-chloräthylthiopyrimidin,
2,4,6-Trichlor-5-β-chloräthylthiopyrimidin,
2,4,6-Tribrom-5-β-chloräthylthiopyrimidin,
2,4,6-Trifluor-5-benzylthiopyrimidin,
2,4,6-Trichlor-5-benzylthiopyrimidin,
2,4,6-Tribrom-5-benzylthiopyrimidin,
2,4,6-Trifluor-5-phenäthylthiopyrimidin,
2,4,6-Trichlor-5-phenäthylthiopyrimidin,
2,4,6-Tribrom-5-phenäthylthiopyrimidin,
2,4,6-Trifluor-5-dichlormethylthiopyrimidin,
2,4,6-Trichlor-5-dichlormethylthiopyrimidin.

Einen weiteren Gegenstand der Erfindung bilden Verbindungen der Formel

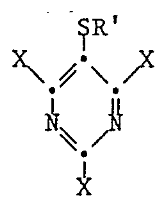

(60),

in denen X Fluor oder Chlor und R′ durch Chlor oder Phenyl substituiertes $C_1$-$C_4$-Alkyl ist.

Bevorzugt sind Verbindungen der Formel (60), in denen X Fluor oder Chlor ist und R′ durch Chlor oder Phenyl substituiertes Methyl oder Aethyl ist.

Ganz besonders bevorzugt sind Verbindungen der Formel (60), in denen X Fluor oder Chlor und R′ Monochlormethyl oder Benzyl ist.

Gegenstand der Erfindung ist ferner ein Verfahren zur Herstellung der Verbindungen der Formel (60), in denen X und R′ die unter Formel (60) angegebenen Bedeutungen haben, welches dadurch gekennzeichnet ist, dass man Barbitursäure mit Verbindungen der Formel

$(R'')_2SO$ (61),

worin R″ gegebenenfalls durch Chlor oder Phenyl substituiertes $C_1$-$C_4$-Alkyl ist, in Gegenwart von Essigsäureanhydrid zu Verbindungen der Formel

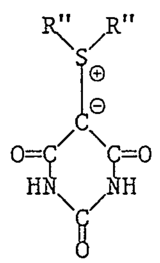

(62),

worin R″ die oben angegebene Bedeutung hat, umsetzt, die Verbindungen der Formel (62) mit Halogenverbindungen des Phosphors in Gegenwart eines Katalysators bei einer Temperatur von 80 bis 180° C umsetzt, und falls R″ unsubstituiertes $C_1$-$C_4$-Alkyl ist eine Chlorierung des Restes R″ durchführt und gegebenenfalls für die Herstellung von Verbindungen der Formel (60), in denen X Fluor ist und R′ die unter Formel (60) angegebene Bedeutung hat, durch Fluorierung mit einem Fluorierungsmittel am Pyrimidinring gebundenes Halogen gegen Fluor austauscht.

Eine Ausführungsform des erfindungsgemässen Verfahrens zur Herstellung von Verbindungen der Formel (60), in denen X Fluor oder Chlor und R′ durch Chlor substituiertes $C_1$-$C_4$-Alkyl ist, besteht darin, das man unsubstituiertes $C_1$-$C_4$-Alkyl eines 2,4,6-Trihalogen-5-$C_1$-$C_4$-alkylthiopyrimidins chloriert, und gegebenenfalls durch Fluorierung mit einem Fluorierungsmittel am Pyrimidinring gebundenes Halogen gegen Fluor austauscht.

Verbindungen der Formel (60), in denen X Chlor und R′ durch Phenyl substituiertes $C_1$-$C_4$-Alkyl ist, können z.B. durch Kondensation von Barbitursäure mit einer Verbindung der Formel (61), in der R″ durch Phenyl substituiertes $C_1$-$C_4$-Alkyl ist, in Gegenwart von Essigsäureanhydrid und anschliessender Umsetzung mit einer Chlorverbindung des Phosphors erhalten werden.

Verbindungen der Formel (60), in denen X Chlor und R′ durch Chlor substituiertes $C_1$-$C_4$-Alkyl ist, können z.B. nach dem oben beschriebenen Verfahren, ausgehend von einer Verbindung der Formel (61), in der R″ unsubstituiertes $C_1$-$C_4$-Alkyl ist, und anschliessender Chlorierung des unsubstituierten $C_1$-$C_4$-Alkyls R″ erhalten werden, wobei die Chlorierung auch direkt ausgehend von einem 2,4,6-Trichlor-5-$C_1$-$C_4$-alkylthiopyrimidin, in welchem das $C_1$-$C_4$-Alkyl unsubstituiert ist, ausgeführt werden kann.

Verbindungen der Formel (60), in denen X Fluor und R′ durch Chlor oder Phenyl substituiertes $C_1$-$C_4$-Alkyl ist, können z.B. ausgehend von den nach den zuvor beschriebenen Verfahren hergestellten Trichlorpyrimidinverbindungen durch Austausch des am Pyrimidinring gebundenen Chlors durch Fluor mit einem Fluorierungsmittel erhalten werden.

Eine bevorzugte Ausführungsform des erfindungsgemässen Verfahrens zur Herstellung von Verbindungen der Formel (60) ist dadurch gekennzeichnet, dass man von Verbindungen der Formel (61), in denen R″ unsubstituiertes oder durch Phenyl substituiertes Methyl oder Aethyl ist, oder gegebenenfalls von 2,4,6-Trichlor-5-methylthio-pyrimidin oder 2,4,6-Trichlor-5-äthyl-thiopyrimidin ausgeht.

Eine besonders bevorzugte Ausführungsform des erfindungsgemässen Verfahrens zur Herstellung von Verbindungen der Formel (60) ist dadurch gekennzeichnet, dass man von Verbindungen der Formel (61), in denen R″ unsubstituiertes oder durch Phenyl substituiertes Methyl ist, oder gegebenenfalls von 2,4,6-Trichlor-5-methylthiopyrimidin ausgeht.

Beispiele für Verbindungen der Formel (61) sind Dimethyl-, Diäthyl-, Dipropyl-, Diisopropyl-, Dibutyl-, Di-tert.-butyl-, Di-sek.-butyl-, Diisobutyl-, Dibenzyl- oder Diphenäthylsulfoxid.

Die Umsetzung von Barbitursäure mit Verbindungen der Formel (61) in Gegenwart von Essigsäureanhydrid erfolgt vorzugsweise in organischen Lösungsmitteln, wie z.B. Acetonitril, bei Temperaturen von z.B. 60 bis 120° C.

Als Halogenverbindungen des Phosphors, mit denen Verbindungen der Formel (62) umgesetzt werden, kommen z.B. Phosphorpentachlorid, -trichlorid oder insbesondere Phosphoroxychlorid in Betracht. Die Umsetzung erfolgt vorzugsweise bei Temperaturen zwischen 100 und 130° C. Als Katalysatoren kommen z.B. Pyridin, Triäthylamin, Tripropylamin, Diäthylanilin oder insbesondere Dimethylanilin in Betracht.

Die Chlorierung des Restes R″, der die oben angegebene Bedeutung hat, erfolgt vorzugsweise mit Chlorierungsmitteln, wie z.B. Chlorgas, in organischen Lösungsmitteln, wie z.B. Tetrachlorkohlenstoff, bei niedrigen Temperaturen, z.B. -40 bis 5° C.

Die Fluorierung erfolgt mit Fluorierungsmitteln, wie z.B. Fluorwasserstoff, Natriumfluorid, Kaliumfluorid oder vorzugsweise einem Kaliumfluorid-Calciumfluorid Gemisch, dessen Verhältnis insbesondere 1:1 ist, in organischen Lösungsmitteln, wie z.B. Sulfolan oder vorzugsweise Acetonitril, bei Temperaturen von z.B. 50 bis 100° C.

Die erfindungsgemässen Reaktivfarbstoffe der Formel (1) eignen sich zum Färben und Bedrucken der verschiedensten Materialien, wie Seide, Leder, Wolle, Polyamidfasern und cellulosehaltiger Fasermaterialien aller Art. Solche Fasermaterialien sind beispielsweise die natürlichen Cellulosefasern, wie Baumwolle, Leinen und Hanf, sowie Zellstoff und regenerierte Cellulose. Die erfindungsgemässen Reaktivfarbstoffe sind auch zum Färben oder Bedrucken von hydroxylgruppenhaltigen Fasern geeignet, die in Mischgeweben enthalten sind, z.B. von Gemischen aus Baumwolle mit Polyesterfasern oder Polyamidfasern.

Die Reaktivfarbstoffe der Formel (1) lassen sich auf verschiedene Weise auf das Fasermaterial applizieren und auf der Faser fixieren, insbesondere in Form von wässrigen Farbstofflösungen und -druckpasten. Sie eignen sich sowohl für das Ausziehverfahren als auch zum Färben nach dem Foulard-Färbeverfahren, wonach die Ware mit wässrigen, gegebenenfalls salzhaltigen Farbstofflösungen imprägniert wird, und die Farbstoffe nach einer Alkalibehandlung oder in Gegenwart von Alkali, gegebenenfalls unter Wärmeeinwirkung fixiert werden. Besonders geeignet sind sie für das sogenannte Kaltverweilverfahren, wonach der Farbstoff zusammen mit dem Alkali auf dem Foulard aufgebracht wird und danach durch mehrstündiges Lagern bei Raumtemperatur fixiert wird. Nach dem Fixieren werden die Färbungen oder Drucke mit kaltem und heissem Wasser, gegebenenfalls unter Zusatz eines dispergierend wirkenden und die Diffusion der nicht fixierten Anteile fördernden Mittels gründlich gespült.

Die erfindungsgemässen Reaktivfarbstoffe zeichnen sich durch hohe Reaktivität, gutes Fixiervermögen und ein gutes Aufbauvermögen aus. Sie können daher nach dem Ausziehfärbeverfahren bei niedrigen Färbetemperaturen eingesetzt werden und erfordern beim Pad-Steam-Verfahren nur kurze Dämpfzeiten. Die Fixiergrade sind hoch, und die nicht fixierten Anteile können leicht ausgewaschen werden.

Die erfindungsgemässen Reaktivfarbstoffe eignen sich zum Färben und Bedrucken von hydroxylgruppen- oder stickstoffgruppenhaltigen Textilmaterialien, insbesondere zum Färben und Bedrucken von Baumwolle und Wolle, und ergeben Färbungen von guten Echtheiten.

Die erfindungsgemässen Reaktivfarbstoffe eignen sich besonders zum Färben von Wolle. Die Färbungen haben vorzügliche Nassechtheiten, insbesondere Pottingechtheiten.

Die nachfolgenden Beispiele dienen zur Erläuterung der Erfindung. Die Temperaturen sind in Celsiusgraden angegeben, Teile sind Gewichtsteile, die Prozentangaben beziehen sich auf Gewichtsprozente, sofern nicht anders vermerkt. Gewichtsteile stehen zu Volumteilen im Verhältnis von Kilogramm zu Liter.

Beispiel 1: 9,18 Teile 2,4,6-Trichlor-5-methylthiopyrimidin werden in 60 Teilen trockenem Tetrachlorkohlenstoff gelöst und in diese Lösung bei -20° innerhalb von 2 Stunden 7,2 Teile Chlorgas eingeleitet. Anschliessend wird die Lösung auf 45° erwärmt, 2 Stunden bei dieser Temperatur gehalten, dann mit Inertgas gespült und destilliert. Man erhält 10,1 Teile der Verbindung der Formel

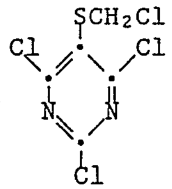

(101)

Beispiel 2: 57 Teile getrocknetes Acetonitril werden vorgelegt. Hierzu werden 46 Teile eines bei 150° am Hochvakuum getrockneten Kaliumfluorid-Calciumfluorid 1:1-Gemisches und 5,02 Teile der gemäss Beispiel 1 hergestellten Verbindung gegeben.

Die Suspension wird unter Rühren 28 Stunden am Rückfluss erhitzt, anschliessend auf Raumtemperatur abgekühlt, filtriert, mit trockenem Acetonitril gewaschen und destilliert. Man erhält 2,28 Teile der Verbindung der Formel

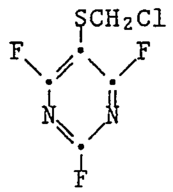

(102)

Beispiel 3: 60 Teile Essigsäure (98-100 %) und 18,9 Teile Essigsäureanhydrid werden vorgelegt. In diese Lösung werden dann 17,42 Teile getrocknete Barbitursäure eingetragen und zu der erhaltenen Suspension 40,76 Teile Dibenzylsulfoxid rasch zugegossen.

Die Suspension wird innerhalb von 2 Stunden auf 90° erwärmt und 4 Stunden bei 90 bis 100° gehalten. Der erhaltene Niederschlag wird bei Raumtemperatur abfiltriert, mit 50 Teilen Wasser, anschliessend mit 40 Teilen Aceton gewaschen und dann im Vakuum bei 40° getrocknet. Man erhält 38,5 Teile eines Zwischenproduktes. 90 Teile Phosphoroxichlorid werden vorgelegt und 6,94 Teile N,N-Dimethylanilin zugetropft. In diese Lösung werden 38,5 Teile des nach obiger Vorschrift erhaltenen Zwischenproduktes eingetragen. Anschliessend wird die Suspension aufgeheizt und 17 Stunden am Rückfluss erhitzt. Nach Abkühlung auf Raumtemperatur wird das überschüssige Phosphoroxichlorid abdestilliert, der dickflüssige Rückstand auf 60 Teile Wasser gegossen und die Temperatur der Suspension bei 25 bei 30° gehalten.

Anschliessend wird die Suspension mit Chloroform extrahiert, das Chloroform abgetrennt und das Reaktionsprodukt im Hochvakuum destilliert. Man erhält 24,58 Teile der Verbindung der Formel

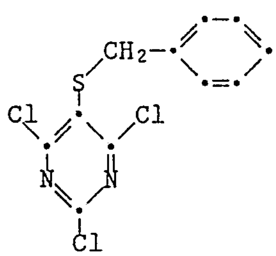

(103)

Beispiel 4: Verfährt man in Beispiel 2 angegeben, verwendet jedoch statt 5,02 Teilen der Verbindung der Formel (101) eine äquimolare Menge der Verbindung der Formel (103), so erhält man die Verbindung der Formel

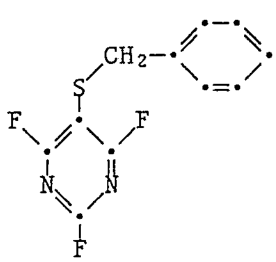

(104)

Beispiel 5: 6,62 Teile des Chromophors der Formel

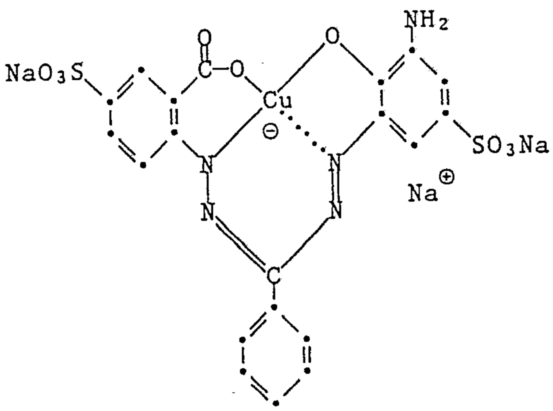

(105)

werden in 50 Teile Wasser eingetragen. Durch Zusatz von Salzsäure wird ein pH-Wert von 7 eingestellt.

Anschliessend werden bei 0 bis 5° 2,1 Teile der Verbindung der Formel

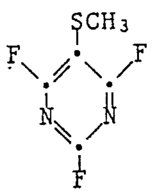

(106),

gelöst in 15 Teilen Aceton, innerhalb von 3 Minuten zugegeben. Der pH-Wert wird durch Zugabe von Natriumhydroxid-Lösung auf einem Wert von 6 bis 7 gehalten. Die Temperatur wird langsam bis Raumtemperatur erhöht, wobei der pH-Wert auf einem Wert von 6 bis 7 gehalten wird.

Anschliessend wird die Reaktionslösung mit Natriumchlorid versetzt, der ausgefallene Farbstoff abfiltriert und bei 60 bis 70° getrocknet.

Man erhält 9,5 Teile eines Farbstoffes, der der Formel

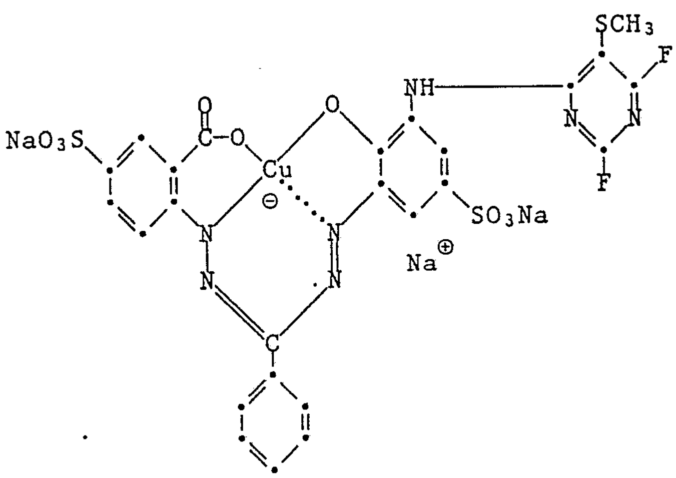

(107)

entspricht. Der erhaltene Farbstoff färbt Wolle in echten blauen Farbtönen.

Beispiele 6 bis 24: Verfährt man wie in Beispiel 5 beschrieben und setzt die in der Tabelle 1 in Spalte 2 angegebenen Chromophore mit den in der Spalte 3 aufgelisteten Pyrimidinverbindungen um, erhält man analoge Farbstoffe, die Wolle oder Baumwolle in der in Spalte 4 angegebenen Nuance färben.

Tabelle 1

| Bsp. | Chromophor | Pyrimidin-Verbindung | Nuance auf Baumwolle oder Wolle |
|---|---|---|---|
| 6 | 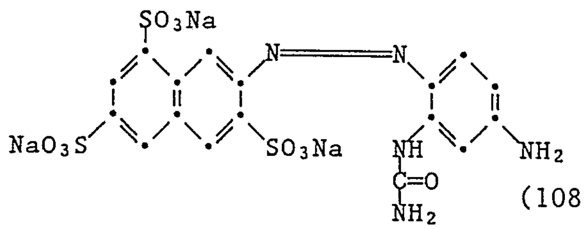 (108) | 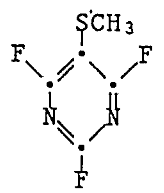 | gelb |
| 7 | 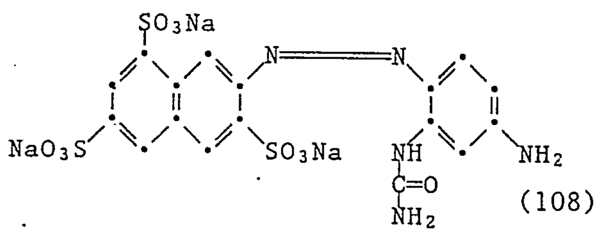 (108) | 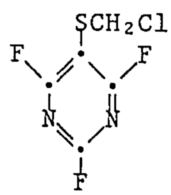 | gelb |
| 8 | 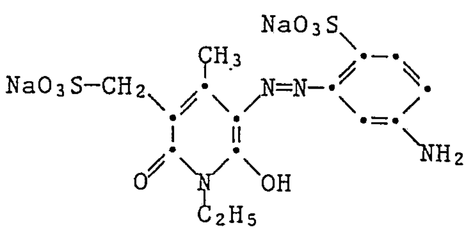 (109) | 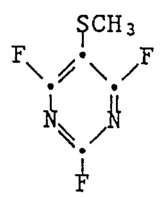 | gelb |
| 9 | 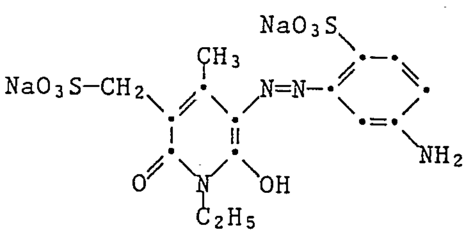 (109) | 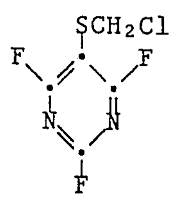 | gelb |
| 10 | 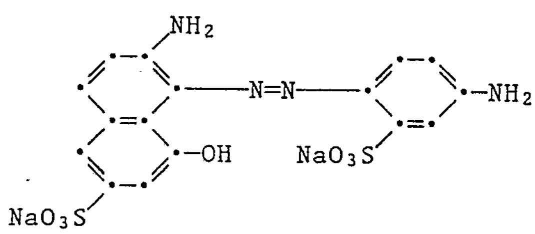 (110) | 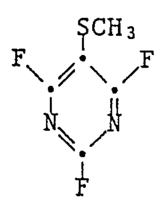 | blaustichig rot |

Tabelle 1 (Fortsetzung)

| Bsp. | Chromophor | Pyrimidin-Verbindung | Nuance auf Baumwolle oder Wolle |
|---|---|---|---|
| 11 | 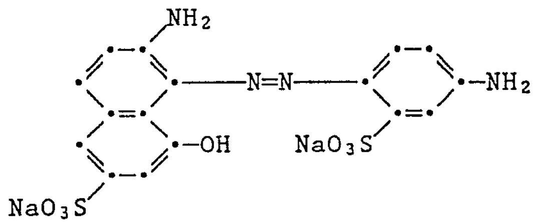 (110) | 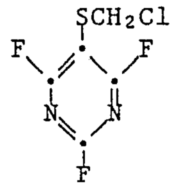 | blaustichig rot |
| 12 | 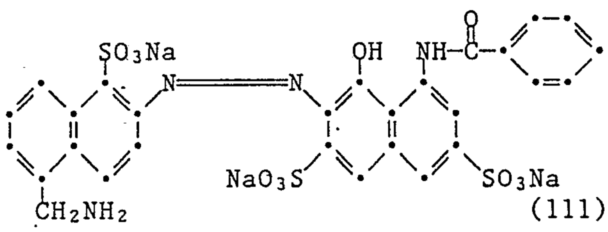 (111) | 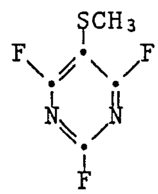 | rot |
| 13 | 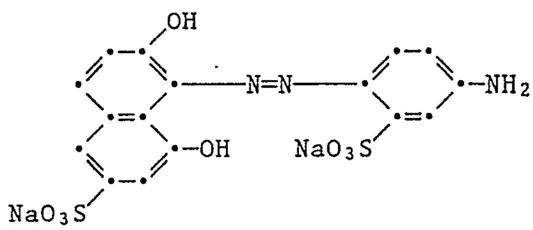 (112) | 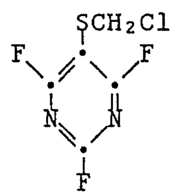 | rot |
| 14 | 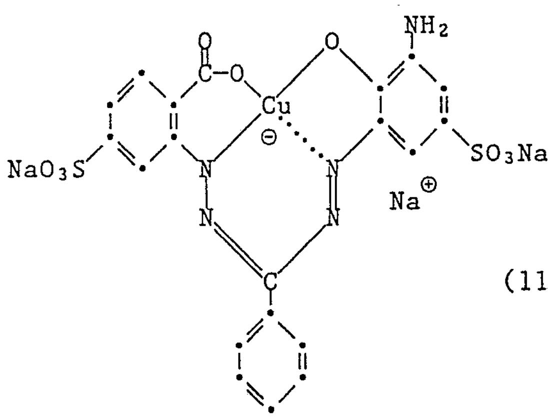 (113) | 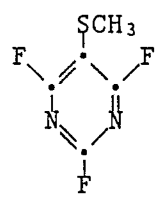 | blau |
| 15 | 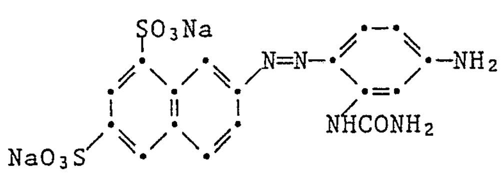 (114) | 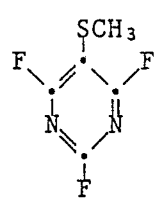 | gelb |

Tabelle 1 (Fortsetzung)

| Bsp. | Chromophor | Pyrimidin-Verbindung | Nuance auf Baumwolle oder Wolle |
|---|---|---|---|
| 16 | 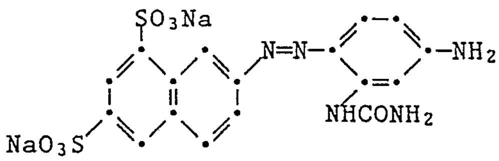 (114) | 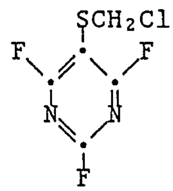 | gelb |
| 17 | 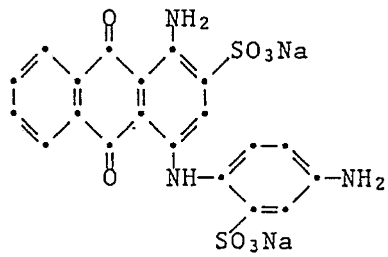 (115) | 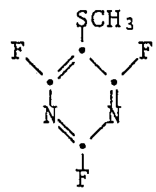 | blau |
| 18 | 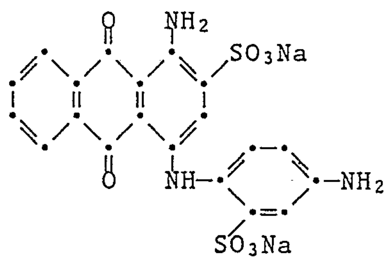 (115) | 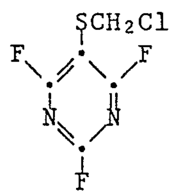 | blau |
| 19 | 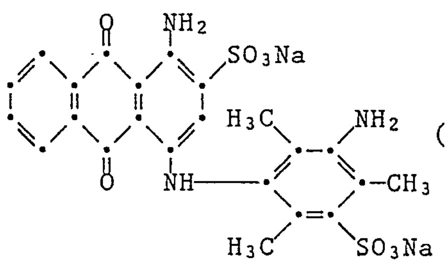 (116) | 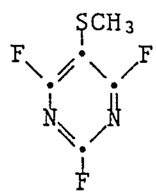 | blau |
| 20 | 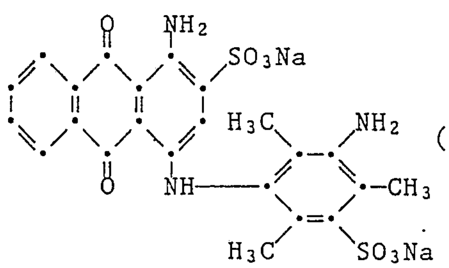 (116) | 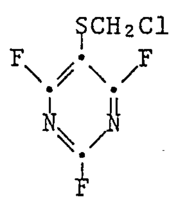 | blau |

Tabelle 1 (Fortsetzung)

| Bsp. | Chromophor | Pyrimidin-Verbindung | Nuance auf Baumwolle oder Wolle |
|---|---|---|---|
| 21 | 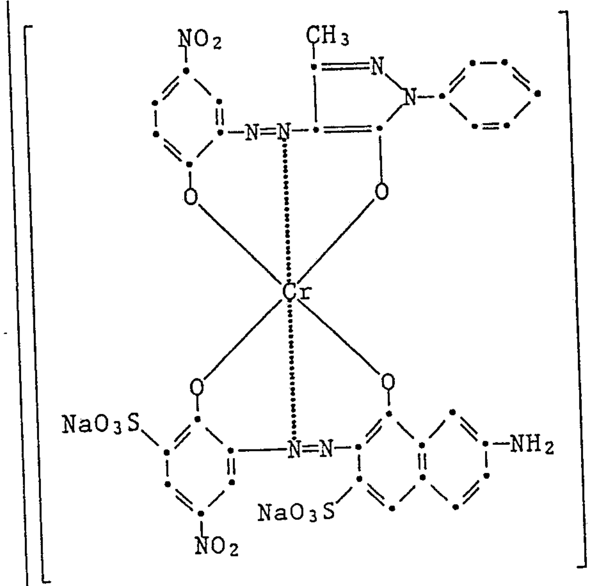 ⊖ Na$^{\oplus}$ (117) | 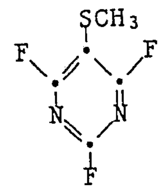 | braun |
| 22 | 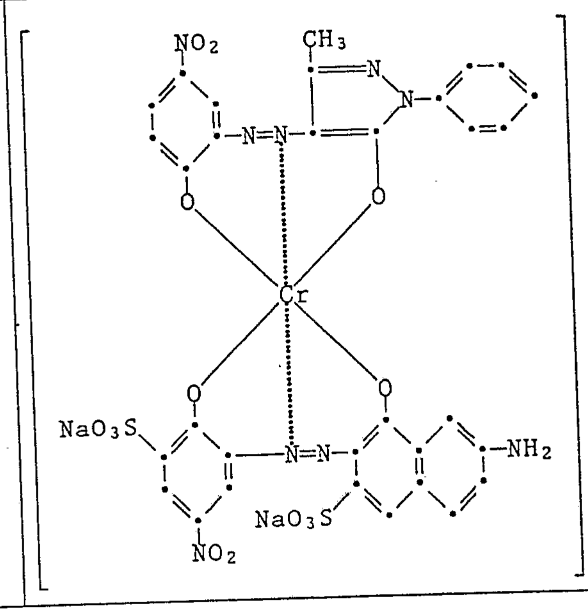 ⊖ Na$^{\oplus}$ (117) | 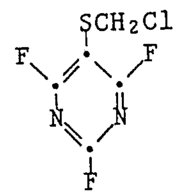 | braun |

Tabelle 1 (Fortsetzung)

| Bsp. | Chromophor | Pyrimidin-Verbindung | Nuance auf Baumwolle oder Wolle |
|---|---|---|---|
| 23 | 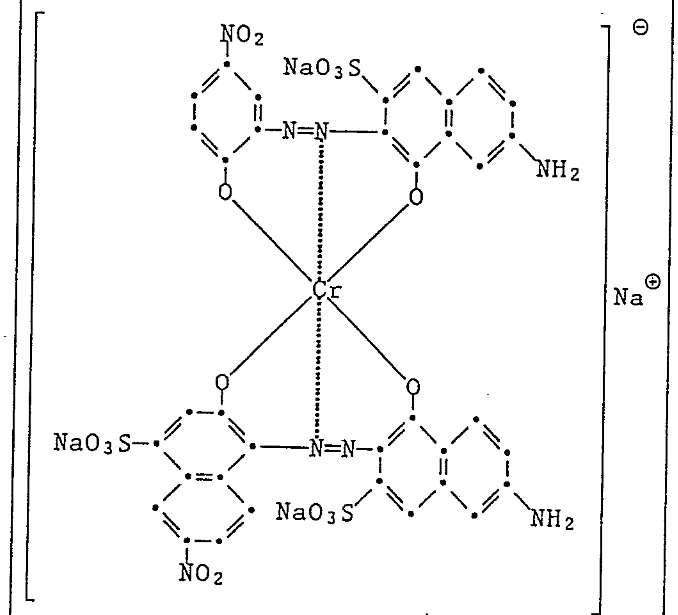 (118) | 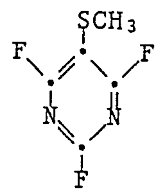 | schwarz |
| 24 | 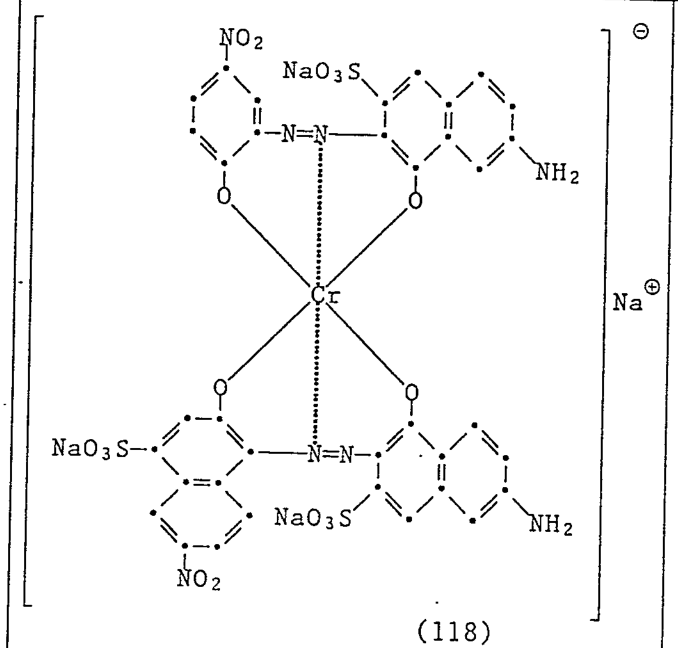 (118) | 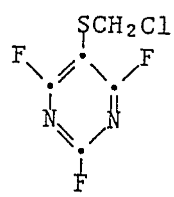 | schwarz |

Beispiel 25: 6,62 Teile des Chromophors der Formel (105) aus Beispiel 5 werden in 50 Teile Wasser eingetragen. Durch Zusatz von Salzsäure wird ein pH-Wert von 7 eingestellt. Anschliessend werden 2,4 Teile der Verbindung der Formel

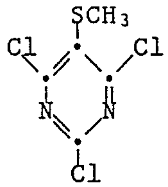

(119),

gelöst in 40 Teilen Aceton, zugegeben. Der pH-Wert wird durch Zugabe von Natriumhydroxid-Lösung auf einem Wert von 7 bis 7,5 gehalten.

Anschliessend wird 8 Stunden bei 40° und einem pH-Wert von 7 bis 7,5 gerührt, die Reaktionslösung filtriert, mit Natriumchlorid versetzt, der ausgefallene Farbstoff abfiltriert, mit Natriumchlorid-Lösung gewaschen und bei 60 bis 70° getrocknet.

Man erhält 10,8 Teile eines Farbstoffes, der der Formel

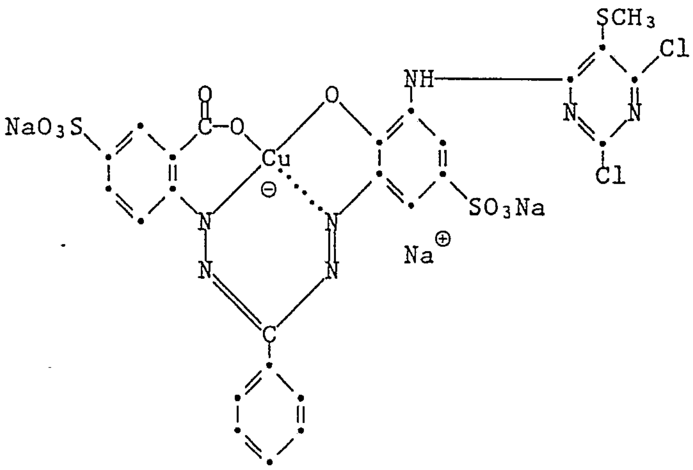

(120)

entspricht. Der erhaltene Farbstoff färbt Wolle in echten blauen Farbtönen.

Beispiele 26 bis 29: Verfährt man wie in Beispiel 25 beschrieben und setzt die in der Tabelle 2 in Spalte 2 angegebenen Chromophore mit den in der Spalte 3 aufgelisteten Pyrimidinverbindungen um, erhält man analoge Farbstoffe, die Wolle oder Baumwolle in der in Spalte 4 angegebenen Nuance färben.

Tabelle 2:

| Bsp. | Chromophor | Pyrimidin-Verbindung | Nuance auf Baumwolle oder Wolle |
|---|---|---|---|
| 26 | 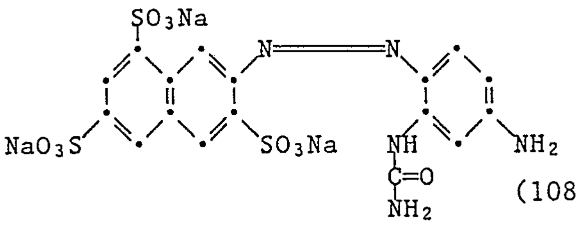 (108) | 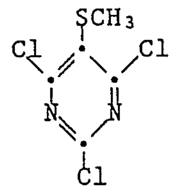 | gelb |
| 27 | 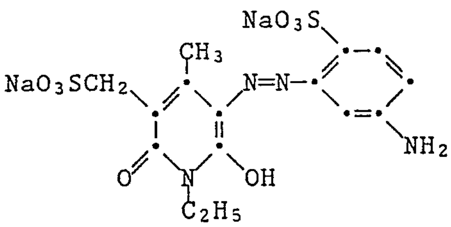 (109) | 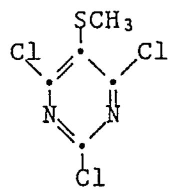 | gelb |
| 28 | 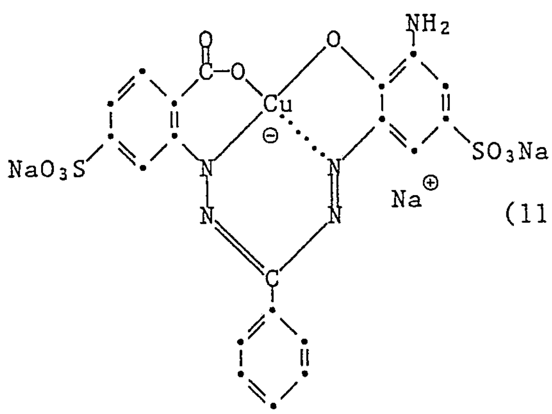 (113) | 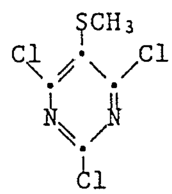 | blau |
| 29 | 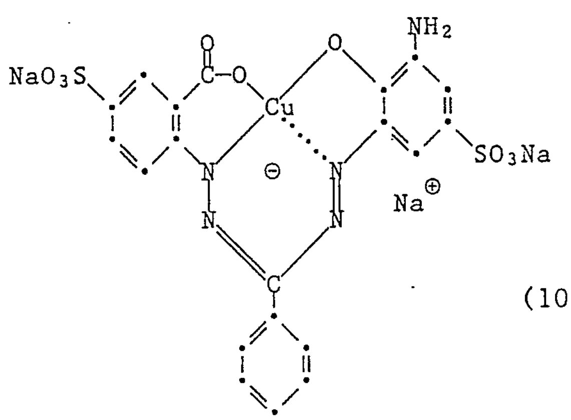 (105) | 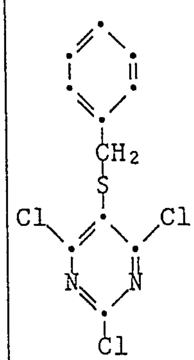 | blau |

Färbebeispiel 1

In 4000 Teilen Wasser von 50° werden nacheinander 4 Teile 80%ige Essigsäure, 2 Teile des Ammoniumsalzes des sauren Schwefelsäureesters des Adduktes aus einem Fettamin (bestehend aus 30 % Hexadecylamin, 25 % Octadecylamin und 45 % Octadecenylamin und 7 Mol Aethylenoxid) sowie 4 Teile Ammoniumsulfat gelöst.

4 Teile des gemäss Beispiel 5 erhaltenen Farbstoffes werden in 100 Teilen Wasser heiss gelöst und dem

obigen Färbebad zugegeben. Dann wird mit 100 Teilen vorgenetzten Wollstrickgarns eingegangen und im Verlaufe von 30 Minuten die Temperatur des Bades von 50 auf 80° erhöht. Nach 20 Minuten Färben bei 80° wird zum Sieden erhitzt und anschliessend 90 Minuten kochend gefärbt. Der Farbstoff zieht fast vollkommen auf das Substrat auf. Nach Abkühlen des Bades auf 80° wird der pH-Wert von etwa 4,5 durch Zugabe von Ammoniaklösung auf bleibend 8,5 erhöht und das Färbegut 20 Minuten bei dieser Temperatur nachbehandelt. Nach gründlichem Spülen mit heissem und kaltem Wasser, Ansäuern mit 1 Teil 80°iger Ameisensäure, Zentrifugieren und Trocknen erhält man ein blau gefärbtes Wollgarn von sehr guter Nass- und Reibechtheit sowie ausgezeichneter Lichtechtheit.

Färbebeispiel 2

Ein filzfest ausgerüstetes Wollgewebe wird mit der nachfolgend beschriebenen Zubereitung getränkt und auf dem Foulard auf eine Feuchtigkeitsaufnahme von 250 % abgequetscht:

| | |
|---|---|
| 4 Teile | Diaprint REG (säurebeständiges Verdickungsmittel), |
| 1 Teil | Sulfaminsäure |
| 0,2 Teile | Thymol |
| 0,2 Teile | Emulgator |
| 94,6 Teile | Wasser |
| 100 Teile | |

Das imprägnierte Material wird anschliessend in einer heizbaren Presse während 3 Minuten bei 100 bis 105° unter einem Druck von etwa 0,5 $kg/cm^2$ mit einem Transferpapier zusammengepresst, welches ein in üblicher Weise mit dem Farbstoff gemäss Beispiel 5 aufgebrachtes Druckmuster trägt. Nach Spülen und Trocknen des Wollgewebes weist dieses ein entsprechendes, blaues Druckmuster von sehr guten Echtheitseigenschaften auf.

Färbebeispiel 3

Ein Flanellgewebe aus chlorierter Wolle wird auf einem Foulard mit nachfolgend beschriebener Farbstoffzubereitung getränkt und auf eine Flüssigkeitsaufnahme von 100 % abgequetscht.

| | |
|---|---|
| 50 Teile | des Farbstoffes gemäss Beispiel 5 |
| 300 Teile | Harnstoff |
| 320 Teile | Solvitose OFA à 4 % (Verdickungsmittel) |
| 10 Teile | einer Mischung von anionaktivem Fettalkoholäthersulfat mit nichtionogenen Netzmitteln |
| 10 Teile | des in Färbebeispiel 1 verwendeten Egalisiermittels |
| 10 Teile | Natrium-metabisulfit |
| 10 Teile | Essigsäure 80%ig |
| 290 Teile | Wasser |
| 1000 Teile | Foulardierflotte. |

Das imprägnierte Gewebe wird anschliessend in aufgerolltem und luftdicht verpacktem Zustand während 48 Stunden bei Raumtemperatur gelagert. Nach Spülen mit kaltem Wasser wird das Material auf frischem Bad mit soviel Ammoniak zu 24 % behandelt, dass ein pH-Wert von 8,5 erreicht wird und während 15 Minuten bei 80° gehalten. Nach Spülen in warmem Wasser wird zum Schluss mit Essigsäure 80%ig abgesäuert und getrocknet. Das Wollgewebe ist in einem vollen Blauton von ausgezeichneten Echtheitseigenschaften gefärbt.

Färbebeispiel 4

Ein Flanellgewebe aus chlorierter Wolle wird auf einem Foulard mit nachfolgend beschriebener Farbstoffzubereitung getränkt und auf eine Flüssigkeitsaufnahme von 100 % abgequetscht:

| | |
|---|---|
| 50 Teile | des Farbstoffes gemäss Beispiel 5 |
| 300 Teile | Solvitose OFA à 40 % (Verdickungsmittel) |
| 20 Teile | einer Mischung von anionaktivem Fettalkoholäthersulfat mit nichtionogenen Netzmitteln |
| 10 Teile | des in Färbebeispiel 1 verwendeten Egalisiermittels |
| 20 Teile | Essigsäure 80%ig |
| 600 Teile | Wasser |
| 1000 Teile | Foulardierflotte. |

Das imprägnierte Gewebe wird anschliessend in einen Dämpfer gebracht und während 20 bis 40 Minuten mit gesättigtem Dampf behandelt. Nach Spülen mit kaltem Wasser wird das Material auf frischem Bad mit soviel 24%iger Ammoniaklösung behandelt, dass ein pH-Wert von 8,5 erreicht wird und während 15 Minuten bei 80° gehalten. Nach Spülen in warmem Wasser wird zum Schluss mit Essigsäure 80%ig abgesäuert und getrocknet. Das Wollgewebe ist in einem vollen Blauton von ausgezeichneten Echtheitseigenschaften gefärbt.

Färbebeispiel 5

In 1000 Teilen Wasser von 50° werden nacheinander 4 Teile 80%ige Essigsäure, 2 Teile des Ammoniumsalzes des sauren Schwefelsäureesters des Adduktes aus einem Fettamin (bestehend aus 30 % Hexadecylamin, 25 % Octadecylamin und 45 % Octadecenylamin und 7 Mol Aethylenoxid) sowie 4 Teile Ammoniumsulfat gelöst.

3 Teile des gemäss Beispiel 5 erhaltenen Farbstoffes werden in 100 Teilen Wasser heiss gelöst und dem obigen Färbebad zugegeben. Ein Zirkulationsfärbeapparat wird mit 100 Teilen vorgenetztem Kammzug in Wickelform beschickt und im Verlaufe von 30 Minuten die Temperatur des Bades von 50 auf 97-99° erhöht, und anschliessend 90 Minuten kochend gefärbt. Der Farbstoff zieht fast vollkommen auf das Substrat auf. Nach Abkühlen des Bades auf 80° wird der pH-Wert von etwa 4,5 durch Zugabe von Ammoniaklösung auf bleibend 8,5 erhöht und das Färbegut bei dieser Temperatur nachbehandelt. Nach gründlichem Spülen mit heissem und kaltem Wasser, Absäuren mit 1 Teil 80%iger Ameisensäure, zentrifugieren und Trocknen erhält man ein blau gefärbtes Färbegut von sehr guter Nassechtheit sowie ausgezeichneter Lichtechtheit.

Färbebeispiel 6

In 1000 Teilen Wasser von 50° werden nacheinander 6 Teile 80%ige Essigsäure, 3 Teile des Ammoniumsalzes des sauren Schwefelsäureesters des Adduktes aus einem Fettamin (bestehend aus 30 % Hexadecylamin, 25 % Octadecylamin und 45 % Octadecenylamin und 7 Mol Aethylenoxid) sowie 6 Teile Ammoniumsulfat gelöst.

3 Teile des gemäss Beispiel 5 erhaltenen Farbstoffes werden in 100 Teilen Wasser heiss gelöst und dem obigen Färbebad zugegeben. Ein Zirkulationsfärbeapparat wird mit 150 Teilen vorgenetzter loser Wolle beschickt und im Verlaufe von 30 Minuten die Temperatur der Flotte von 50° auf 97-99° erhöht, und anschliessend 90 Minuten kochend gefärbt. Der Farbstoff zieht fast vollkommen auf das Substrat auf. Nach Abkühlen der Flotte auf 80° wird der pH-Wert von etwa 4,5 durch Zugabe von Ammoniaklösung auf bleibend 8,5 erhöht und das Färbegut 20 Minuten bei dieser Temperatur nachbehandelt. Nach gründlichem Spülen mit heissem und kaltem Wasser, Absäuren mit 1 Teil 80%iger Ameisensäure, Zentrifugieren und Trocknen erhält man ein faseregal blau gefärbtes Substrat mit guter Nass- und Reibechtheit sowie ausgezeichneter Lichtechtheit.

Färbebeispiel 7

2 Teile des gemäss Beispiel 6 erhaltenen Farbstoffes werden in 400 Teilen Wasser gelöst; dazu gibt man 1500 Teile einer Lösung, die pro Liter 53 g Natriumchlorid enthält. In diese Färbebad geht man bei 40° C mit 100 Teilen eines Baumwollgewebes ein. Nach 45 Minuten werden 100 Teile einer Lösung, die pro Liter 16 g Natriumhydroxyd und 20 g kalziniertes Soda enthält, zugegeben. Die Temperatur des Färbebades wird weitere 45 Minuten bei 40° C gehalten. Danach wird die gefärbte Ware gespült, während einer Viertelstunde mit einem nichtionogenen Waschmittel kochend geseift, nochmals gespült und getrocknet.

**Patentansprüche**

1. Reaktivfarbstoffe der Formel

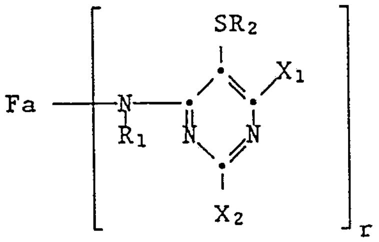

(1),

worin Fa der Rest eines organischen Farbstoffes der Monoazo- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbimid-Reihe, $X_1$ und $X_2$ unabhängig voneinander je Halogen, $R_1$ Wasserstoff oder gegebenenfalls substituiertes $C_1$-$C_4$-Alkyl, $R_2$ gegebenenfalls substituiertes $C_1$-$C_4$-Alkyl und r die Zahl 1 oder 2 ist.

2. Reaktivfarbstoffe gemäss Anspruch 1, dadurch gekennzeichnet, dass r die Zahl 1 ist.

3. Reaktivfarbstoffe gemäss einem der Ansprüche 1 oder 2, dadurch gekennzeichnet, dass $X_1$ und $X_2$ unabhängig voneinander je Chlor oder Fluor sind.

4. Reaktivfarbstoffe gemäss einem der Ansprüche 1 oder 2, dadurch gekennzeichnet, dass $X_1$ und $X_2$ jeweils Chlor oder Fluor bedeuten.

5. Reaktivfarbstoffe gemäss einem der Ansprüche 1 bis 4, dadurch gekennzeichnet, dass $R_1$ Methyl, Aethyl oder insbesondere Wasserstoff ist.

6. Reaktivfarbstoffe gemäss einem der Ansprüche 1 bis 5, dadurch gekennzeichnet, dass $R_2$ gegebenenfalls durch Halogen, insbesondere Chlor, oder Phenyl substituiertes Methyl oder Aethyl ist.

7. Reaktivfarbstoffe gemäss einem der Ansprüche 1 bis 5, dadurch gekennzeichnet, dass $R_2$ Methyl, Monochlormethyl oder Benzyl ist.

8. Reaktivfarbstoffe gemäss einem der Ansprüche 1 bis 7, dadurch gekennzeichnet, dass Fa unsubstituiert oder durch gegebenenfalls durch Sulfo substituiertes $C_1$-$C_4$-Alkyl, $C_1$-$C_4$-Alkoxy, Phenyl, $C_1$-$C_4$-Alkanoylamino, Benzoylamino, Amino, unsubstituiertes oder im Alkylteil durch -OH, $-OCOCH_3$, $-OSO_3H$-, -CN oder Halogen substituiertes N-Mono- oder N,N-Di-$C_1$-$C_4$-Alkylamino, gegebenenfalls durch Sulfo und/oder $C_1$-$C_4$-Alkyl substituiertes Phenylamino, Mono- oder Di-Sulfobenzylamino, $C_1$-$C_4$-Alkoxycarbonyl, $C_1$-$C_4$-Alkylsulfonyl, Trifluormethyl, Nitro, Cyano, Halogen, Carbamoyl, N-Mono- oder N,N-Di-$C_1$-$C_4$-Alkylcarbamoyl, Sulfamoyl, N-Mono-oder N,N-Di-$C_1$-$C_4$-Alkylsulfamoyl, N-Mono- oder N,N-Di-(β-hydroxyäthyl)-sulfamoyl, N-Phenylsulfamoyl, Hydroxy, Carboxy, Sulfo, Sulfomethyl und/oder Ureido substituiert ist.

9. Reaktivfarbstoffe gemäss einem der Ansprüche 1 bis 8, dadurch gekennzeichnet, dass Fa für den Rest eines organischen Farbstoffes der Mono-oder Polyazo-, Metallkomplexazo-, Anthrachinon-, Phthalocyanin-, Formanzan-oder Dioxazinreihe, welcher durch einen oder mehrere der im Anspruch 8 genannten Reste substituiert ist, steht.

10. Reaktivfarbstoffe der Formel (1) gemäss Anspruch 1, worin Fa für den Rest eines organischen Farbstoffes der Mono- oder Polyazo-, Metallkomplexazo-, Anthrachinon-, Phthalocyanin-, Formazan- oder Dioxazinreihe, welcher eine oder mehrere Sulfogruppen trägt und gegebenenfalls durch einen oder mehrere der im Anspruch 8 genannten Reste weitersubstituiert ist, steht, $R_1$ Wasserstoff oder unsubstituiertes oder durch Halogen, Hydroxy, Cyano, Carboxy, Sulfo, Sulfato, $C_1$-$C_4$-Alkoxycarbonyl oder $C_1$-$C_4$-Alkoxy substituiertes $C_1$-$C_4$-Alkyl darstellt, $X_1$ und $X_2$ unabhängig voneinander je Chlor oder Fluor, $R_2$ gegebenenfalls durch Halogen, insbesonders Chlor, oder Phenyl substituiertes Methyl oder Aethyl und r die Zahl 1 ist.

11. Reaktivfarbstoffe gemäss Anspruch 10 der Formel

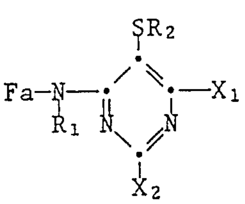

(2),

worin Fa die im Anspruch 10 angegebene Bedeutung hat, $R_1$ Wasserstoff, Methyl oder Aethyl ist, $X_1$ und $X_2$ jeweils Chlor oder Fluor bedeuten und $R_2$ Methyl, Monochlormethyl oder Benzyl ist.

12. Reaktivfarbstoffe gemäss Anspruch 1 der Formel

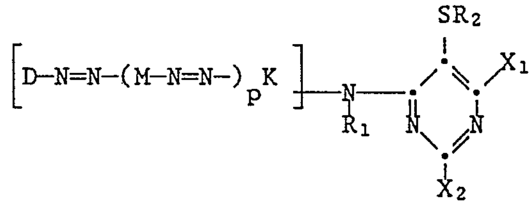

(3)

oder

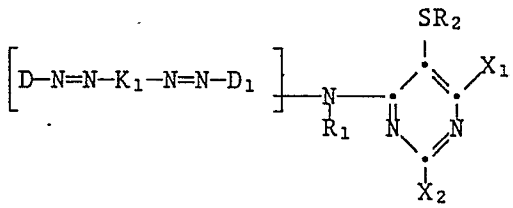

(4),

worin $R_1$, $R_2$, $X_1$ und $X_2$ die im Anspruch 1 angegebenen Bedeutungen haben, D und $D_1$ unabhängig voneinander eine Diazokomponente der Benzol- oder Naphthalinreihe oder der heterocyclischen Reihe darstellen, M eine Mittelkomponente der Benzol oder Naphthalinreihe ist, K für eine Kupplungskomponente der Benzol- oder Naphthalinreihe oder der heterocyclischen Reihe steht, $K_1$ der Rest einer Kupplungskomponente der Aminonaphtholsulfonsäure-Reihe ist und p die Zahl 0 oder 1 bedeutet.

13. Reaktivfarbstoffe gemäss Anspruch 1 der Formeln

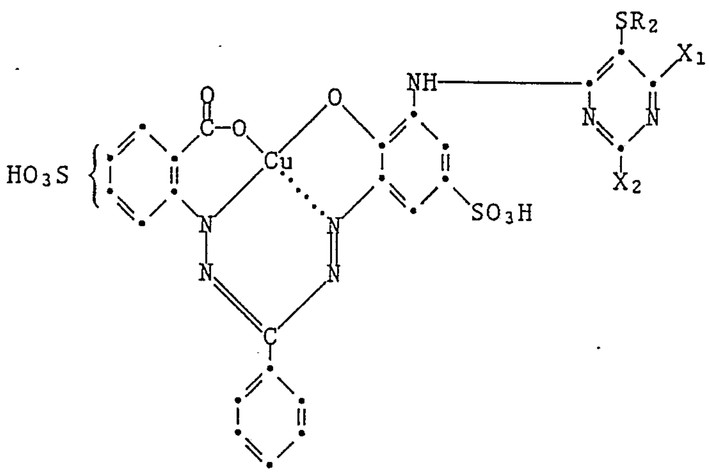

(47)

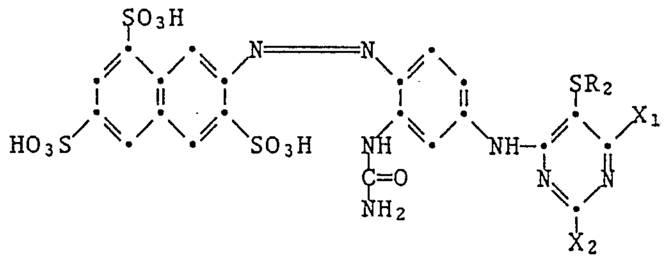

(48)

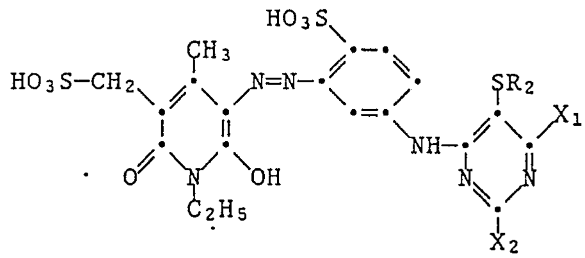

(49)

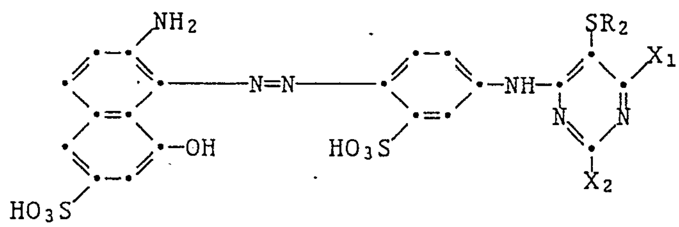

(50)

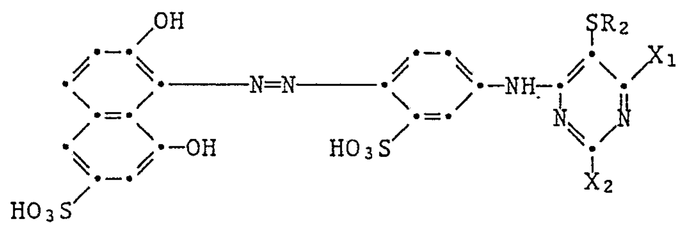

(51)

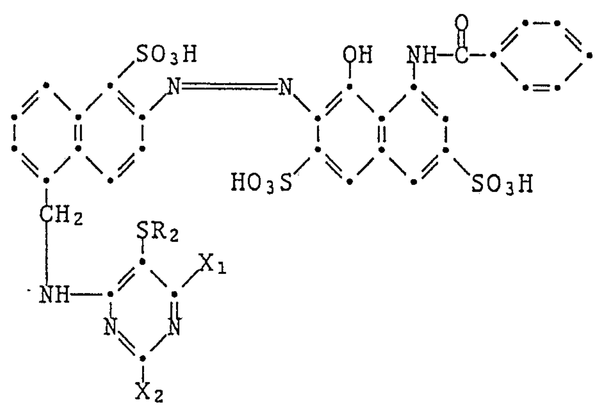

(52),

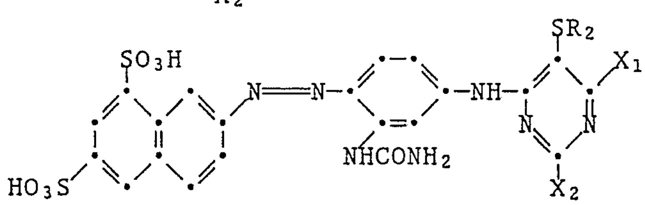

(53),

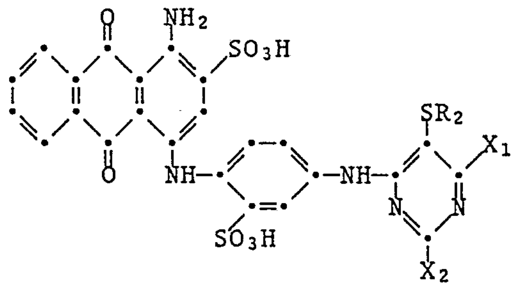

(54),

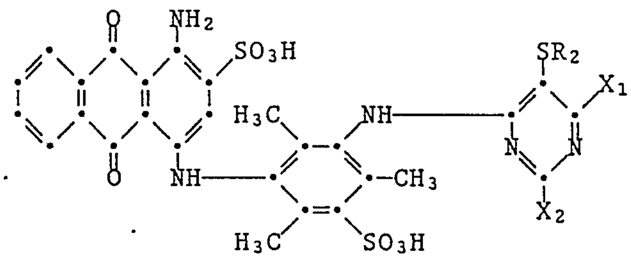

(55),

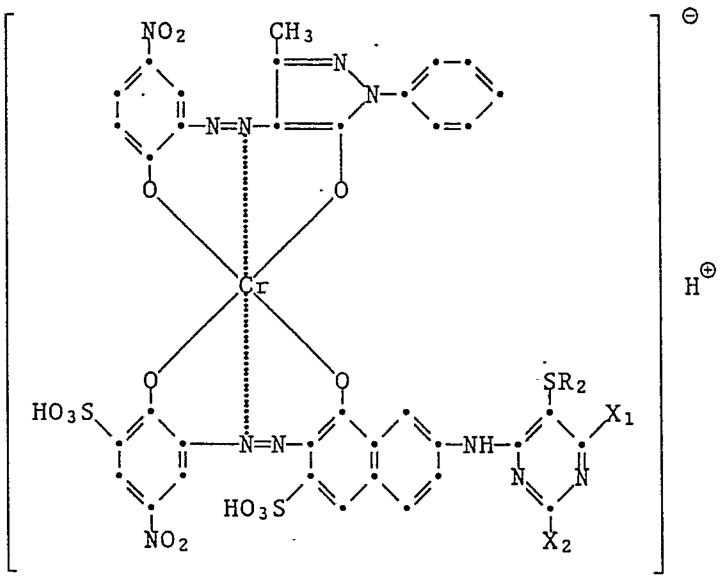

(56)

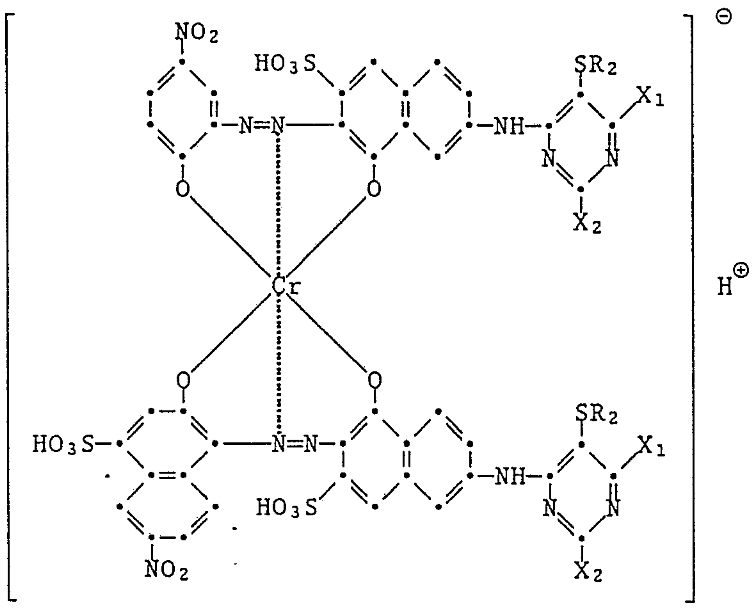

(57),

worin $R_2$, $X_1$ und $X_2$ die im Anspruch 1 angegebenen Bedeutungen haben.

14. Verfahren zur Herstellung von Reaktivfarbstoffen der Formel (1), dadurch gekennzeichnet, dass man organische Farbstoffe der Formel

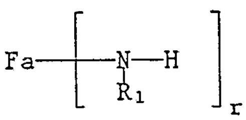

(58)

oder Farbstoffvorprodukte mit mindestens einem Aequivalent eines Pyrimidinderivats der Formel

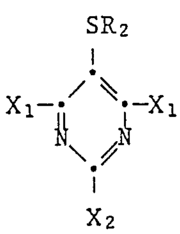

(59)

zu einem Reaktivfarbstoff der Formel (1) kondensiert, wobei Fa, $R_1$, $R_2$, $X_1$, $X_2$ und r die in Anspruch 1 angegebenen Bedeutungen haben.

15: Verbindungen der Formel

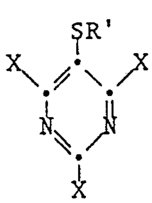

(60),

in denen X Fluor oder Chlor und R′ durch Chlor oder Phenyl substituiertes $C_1$-$C_4$-Alkyl ist.

16. Verwendung der Reaktivfarbstoffe gemäss Anspruch 1 zum Färben und Bedrucken von hydroxylgruppen- oder stickstoffgruppenhaltigen Textilmaterialien.

17. Verwendung der Reaktivfarbstoffe gemäss Anspruch 1 zum Färben und Bedrucken von Baumwolle und Wolle.